# EUROPEAN PATENT SPECIFICATION

(11) **EP 2 254 999 B1**
(45) Date of publication and mention of the grant of the patent: **22.05.2019**
(21) Application number: 09707888.5
(22) Date of filing: 06.02.2009
(51) Int. Cl.: C12N 15/10, B03D 1/18

(54) **METHOD AND USE OF DEVICE FOR PRODUCING BIOMOLECULES**
VERFAHREN UND VERWENDUNG EINER VORRICHTUNG ZUR HERSTELLUNG VON BIOMOLEKÜLEN
PROCÉDÉ ET UTILISATION D'UN DISPOSITIF POUR LA PRODUCTION DE BIOMOLÉCULES

(30) Priority: 08.02.2008 EP 08151209
(43) Date of publication of application: 01.12.2010
(73) Proprietor: Boehringer Ingelheim RCV GmbH & Co KG, 1120 Wien (AT)
(72) Inventor: URTHALER, Jochen, 55216 Ingelheim Am Rhein (DE); ASCHER, Christine, 55216 Ingelheim Am Rhein (DE); BUCHELI, Daniel, 55216 Ingelheim Am Rhein (DE)
(74) Representative: Simon, Elke Anna Maria
(86) International application number: PCT/EP2009/051370
(87) International publication number: WO 2009/098284

(56) References cited:
- EP-A- 1 593 741
- WO-A-92/17260
- WO-A-03/070942
- WO-A-2004/024283
- WO-A-2004/085643
- WO-A-2004/108260
- DE-A1- 4 335 996
- US-A- 5 382 358
- URTHALER J ET AL: "Application of monoliths for plasmid DNA purification - Development and transfer to production" JOURNAL OF CHROMATOGRAPHY, ELSEVIER SCIENCE PUBLISHERS B.V. AMSTERDAM, NL, vol. 1065, no. 1, 11 February 2005 (2005-02-11), pages 93-106, XP004750096 ISSN: 0021-9673
- URTHALER JOCHEN ET AL: "Automated alkaline lysis for industrial scale cGMP production of pharmaceutical grade plasmid-DNA." JOURNAL OF BIOTECHNOLOGY 30 JAN 2007, vol. 128, no. 1, 30 January 2007 (2007-01-30), pages 132-149, XP002485653 ISSN: 0168-1656 cited in the application
- THEODOSSIOU I ET AL: "Methods of enhancing the recovery of plasmid genes from neutralised cell lysate" BIOPROCESS ENGINEERING, SPRINGER VERLAG, DE, vol. 20, no. 2, 1 February 1999 (1999-02-01), pages 147-156, XP002338362 ISSN: 0178-515X cited in the application
- CICCOLINI L A S ET AL: "Rheological properties of chromosomal and plasmid DNA during alkaline lysis reaction" BIOPROCESS ENGINEERING, SPRINGER VERLAG, DE, vol. 21, no. 3, 1 September 1999 (1999-09-01), pages 231-237, XP002314371 ISSN: 0178-515X
- URTHALER JOCHEN ET AL: "Improved downstream process for the production of plasmid DNA for gene therapy." ACTA BIOCHIMICA POLONICA 2005, vol. 52, no. 3, 2005, pages 703-711, XP002485654 ISSN: 0001-527X

## Description

### Field of invention

The present invention relates to the field of producing biomolecules, in particular polynucleotides like plasmid DNA. In particular, the present invention relates to a gentle clarification step by an advanced floatation method mediated by generation of gas bubbles without gas/air injection. The present invention is particularly suited for a method on an industrial scale that includes cell lysis under alkaline conditions followed by neutralization and subsequent clarification of the cell lysate, whereas all these steps are carried out in a completely continuous mode.

### Background of the invention

The advances in molecular and cell biology in the last quarter of the 20^{th} and in the beginning of the 21^{st} century have led to new technologies for the production of recombinant biomolecules (biopolymers). This group of macromolecules includes e.g. proteins, nucleic acids and polysaccharides. They are increasingly used in human health care, in the areas of diagnostics, prevention and treatment of diseases.

Recently some of the most revolutionary advances have been made with polynucleotides in the field of diagnostics, gene therapy and nucleic acid vaccines. Common to these applications is the introduction of DNA, in particular extrachromosomal DNA, or RNA into cells with the aim of a diagnostic, therapeutic or prophylactic effect.

Representative members of polynucleotides are messenger RNA (mRNA), transfer RNA (tRNA) and ribosomal RNA (rRNA), genomic DNA (gDNA) or chromosomal DNA (cDNA), and plasmid DNA (pDNA). These macromolecules can be single- or doublestranded.

Polynucleotides are sensitive to enzymatic degradation (DNases and RNases) and shear forces, depending on their size and shape. Especially chromosomal DNA, in its denatured and entangled form, is highly sensitive to mechanical stress, resulting in fragments with similar properties to pDNA. This becomes more and more critical with the duration of the shear force exposure (Ciccolini LAS, Shamlou PA, Titchener-Hooker N, Ward JM, Dunnill P (1998) Biotechnol Bioeng 60:768; Ciccolini LAS, Shamlou PA, Titchener-Hooker N (2002) Biotechnol Bioeng 77:796).

Plasmids (pDNA) are double stranded extrachromosomal circular polynucleotides. Most of the pharmaceutical plasmids are in the size range of 3 - 10 kbp, which corresponds to a molecular mass of 2 x 10⁶ - 7 x 10⁶ with a radius of gyration of 100 nm and higher (Tyn M, Gusek T (1990) Biotech. Bioeng. 35:327). In some cases polycistronic/polyvalent plasmids larger than 20 kbp, which encode for several different proteins, have been reported (Müller PP, Oumard A, Wirth D, Kröger A, Hauser H, in: Schleef M (2001) Plasmids for Gene Therapy and Vaccination, Wiley-VCH, Weinheim, p 119). Different topological forms of pDNA can be distinguished. The supercoiled (sc) or covalently closed circular (ccc) form is considered as most stable for therapeutic application and is therefore the desired form. The other topological pDNA forms are derived from the ccc form by either single strand nick (open circular or oc) or double strand nick (linear) or result from conjugation. Breakage of the strands can be caused by physical, chemical or enzymatic activity. For therapeutic use the percentage of ccc form is the main-parameter for assessing the quality of the pDNA preparation.

The increasing number of clinical trials in the field of gene therapy and genetic vaccination reflect the potential advantages of pDNA. Thus it is also observed that the demand of pharmaceutical grade pDNA produced according to cGMP rules increases continuously. Forecasts confirm that especially for the market supply with pDNA-based vaccines many grams or even several kilograms purified pDNA per year will be required. Thus there is a demand for processes that can be performed on an industrial scale. These production processes must fulfill regulatory requirements (e.g. FDA, EMEA), should be economically feasible and must be productive and robust.

In the past, the majority of biotechno logical production processes have been developed for manufacturing of purified recombinant proteins. Due to the differences in the physicochemical properties between polynucleotides and proteins, these methods cannot easily be adapted for the production of polynucleotides. Manufacturers who established production processes based on traditional lab-scale protocols more and more face severe limitations regarding productivity, scaleability and costs of goods (COGS). Thus, there is a need for methods that are applicable to polynucleotides, in particular for production of pDNA on a manufacturing scale.

In brief, a process for producing recombinant biomolecules, which are not secreted by the host, in particular pDNA and large proteins, follows the steps of:
a) fermentation (cultivation of cells that carry the biomolecule of interest and optionally harvesting the cells from the fermentation broth),
b) disintegration of the cells (release of the bio molecule of interest from the cells),
c) isolation and purification (separation of the biomolecule of interest from impurities).

These steps are more specifically characterized for the production of polynucleotides, in particular for the production of pDNA, as described below.

Currently, *E.coli* is the most commonly host used for pDNA production. Other bacteria, yeasts, mammalian and insect cells may also be used as host cells in the fermentation step. Selection of a suitable host strain, a well-defined culture medium applied in a high cell density process and maintenance of a high plasmid copy number are of major importance for the pDNA quality and crucial for a robust economic process (Werner RG, Urthaler J, Kollmann F, Huber H, Necina R, Konopitzky K (2002) Contract Services Europe, a supplement to Pharm. Technol. Eur. p. 34).

After fermentation, the cells are usually harvested, mostly by means of centrifugation. The harvested wet biomass is resuspended in an appropriate buffer. Before final isolation of the polynucleotide of interest from impurities (host related: e.g. proteins, gDNA, RNA and endotoxins; product related: e.g. undesired polynucleotide isoforms; process related: e.g. residual compounds of the fermentation medium), the cells need to be processed, either directly or after freezing and thawing. Alternatively to harvesting and resuspending the cells before further processing, the fermentation broth per se may be subject to further processing (WO 97/29190).

Processing starts with disintegration of the cells (polynucleotide release) and ends with the recovery of the clarified solution containing the polynucleotide of interest. During the subsequent isolation of the polynucleotide of interest (by e.g. column chromatography, ultradiafiltration, extraction or precipitation) it has to be separated from the impurities. Disintegration of the cells can be achieved by physical, chemical or enzymatic methods. Usually, disintegration and release of the polynucleotide of interest from bacterial cells is performed by alkaline lysis as described by Birnboim and Doly (Birnboim HC, Doly J (1979) Nucl Acids Res 7: 1513).

The disintegration/release process disclosed therein can be divided into two steps, the first one being the intrinsic cell disintegration or lysis step and the second one being the neutralization step.

During alkaline lysis, cells are subjected to an alkaline solution (preferably NaOH) in combination with a detergent (preferably sodium dodecyl sulfate (SDS)). In this environment, the cell wall structures are destroyed thereby releasing the polynucleotide of interest and other cell related compounds. Finally, the solution is neutralized by addition of a solution of an acidic salt, preferably an acetate, in particular potassium acetate (KAc) or sodium acetate (NaAc). During neutralization cell debris, proteins as well as gDNA are co-precipitated with dodecyl-sulfate by formation of a floccose precipitate (Levy MS, Collins IJ, Yim SS, et al. (1999) Bioprocess Eng 20:7).

In the next step that follows alkaline lysis and neutralization, the precipitate has to be separated from the plasmid containing solution. This step is, in the meaning of the present invention, termed "clarification step".

Regarding the clarification step, centrifugation on fixed angle rotors is the most frequently used method employed on laboratory and pre-preparative scales (Ferreira GNM, Cabral JMS, Prazeres DMF (1999) Biotechnol Prog 15:725). For lysate amounts usually handled in bottles a clearer liquid phase is separating from a large phase of floating flocs and some descending (non-floating) precipitate after a while. Only the clearer liquid phase is sucked off and filtered. Otherwise the big floc volume would immediately block the filter used. The filtration process is even more hampered due to the increased amount of flocs in the clearer phase when the flocs were disrupted due to mechanical stress, resulting in reduced floatation tendency and poor phase separation. In general the layer of floating floes is not very compact and therefore the percentage of the lower clearer phase is about 50-70 % of the whole volume (clearer phase and floc phase). Since the fluid in the floc phase contains residual plasmid DNA (Theodossiou I, Collins IC, Ward JM, Thomas ORT, Dunnhill P (1997) Bioproc Eng 16:175), high losses of up to 40% have to be taken into account (Urthaler J, Ascher C, Wöhrer H, Necina R (2007) J Biotechnol 128:132). Further more, strong adsorption of (poly)nucleotides such as pDNA to various filter-media has to be considered (Theodossiou I, Collins IJ, Ward JM, Thomas ORT, Dunnhill P (1997) Bioprocess Eng 16:175; Theodossiou I, Thomas ORT, Dunnhill P (1999) Bioprocess Eng 20: 147). Often bulk filter materials or bag filters are used for clarifying the lysate. Since these materials are either not certified or not scalable, they are not applicable for the production of pharmaceutical-grade plasmids on a manufacturing scale. More recent technologies utilize expanded bed adsorption (EBA), which allows removal of precipitated material while capturing the desired product (Chase HA (1994) Trends Biotechnol 12: 296). However, it has to be taken into account that due to the large diameter of the aggregated flocs, generated during neutralization, pre-clarification prior to EBA is essential (Ferreira GNM, Cabral JMS, Prazeres DMF (2000) Bioseparation 9:1; Varley DL, Hitchkock AG, Weiss AME, et al. (1998) Bioseparation 8:209).

There have been several attempts to develop improved technologies for each of the above-described steps, which are often operated in non-continuous open systems that bear the risk of possible contamination. The process steps are not automated and the results therefore user-dependent. The methods and devices used are therefore not suitable for the production of pharmaceutical grade polynucleotides on a manufacturing scale. The only way to handle large pDNA-amounts for such processes is multiplying the devices, e.g. to run them in parallel.

In view of further purification of the polynucleotide of interest, it is often necessary to adjust the parameters of the solution (such as salt composition, conductivity, pH-value) to guarantee binding of the desired polynucleotide on a resin (this adjustment step is, in the meaning of the present invention, termed "conditioning step"). Subsequently, the solution is subjected to the first chromatographic step (capture step).

The limiting factor, which is probably most difficult to overcome is the clarification of the lysate. To obtain a cleared lysate the precipitated material has to be separated from the polynucleotide containing solution. Conventionally this clarification step is carried out in a batchwise mode using techniques known in the art like filtration or centrifugation (e.g. US 2001/0034435, WO 02/04027). Most commonly, the filters are depth filters (WO 00/09680). Other filter means for macrofiltration are macroporous diaphragms consisting of e.g. compressed gauze or an equivalent filter material (EP 0376080). According to some protocols, filtration is carried out in presence of a filter aid (WO 95/21250, WO 02/057446, US 2002/0012990). WO 96/21729 discloses a method that contains a filtration step using diatomaceous earth after a centrifugation step, thereby achieving an additional effect of reducing the RNA content. Furthermore, combinations of a membrane filter with a loose matrix (glass, silica-gel, anion exchange resin or diatomaceous earth), which concurrently act as carrier for DNA, have been described in EP 0814156. According to WO 96/08500, WO 93/11218, EP 0616638 and EP 0875271, clarification is achieved by a device, whose filtration part may consist of different materials (e.g. glass, silica-gel, aluminum oxide) in the form of loose particles, layers or filter plates (especially with an asymmetric pore size distribution). The flux through the filter is accomplished by gravitation, vacuum, pressure or centrifugation. Another option to achieve separation of flocs and lysate is described in WO 2004/024283 and WO 2004/108260. Therein, one of the buffers/solutions used in the process prior or during neutralization is mixed with a controllable stream of gas introduced via a gas port. Fine distribution of the gas is accomplished by a sparge stone, which is in fluid communication with the solution/suspension and which provides small gas bubbles of a certain size. The small gas bubbles get attached to the precipitate generated during neutralization. The neutralised lysed cell solution is collected in a tank and hold for a certain time, which is needed to let the majority of floes float (mediated by the attached gas bubbles). Afterwards the lower lysate phase is filtered batch wise via a set of filters. In another setup the lysate floc mixture (obtained with or without gas injection) is collected in a tank, which is designed in a way to additionally allow application of under-pressure (vacuum) above the floc/lysate phase (WO 03/070942, WO 2004/108260). The vacuum improves the floatation of the flocs and directs them to the top of the collection tank. Both methods perform clarification in a non-continuous mode and need additional filtration steps for clarification. Centrifugation as a continuous clarification method (e.g. disc stack centrifuge or decanting centrifuge) is disclosed in WO 99137750 and WO 96/02658. Also combinations of centrifugation followed by filtration are described for the clarification purpose (WO 02/26966, WO 96/02658). EP1593741 describes an alternative method of producing a microbial clear lysate containing nucleic acid material useful for pharmaceutical applications comprising breaking up microorganisms and removing cellular components by flotation.

The above-described clarification methods are usually carried out after the material has been incubated with the neutralization buffer for a certain period of time. This does not allow continuous connection with the foregoing and following steps and is limited in scale.

Apart from this, filtration techniques are often carried out in open devices with the risk of possible contamination. Since any material used in a cGMP process must be validated, additional filter aids although improving the performance of the filtering process, are usually avoided. Yet another disadvantage of the clarification methods known in the art is a long contact time of flocs and lysate (before/during separation), which should be avoided in order to reduce the risk of impurity redissolution and enzymatic degradation.

In general, conventional filters have limited capacity and are soon blocked by the large amount of voluminous flocs. In addition, a constant flux over the precipitate that is retained by the material may result in destruction of the flocs and re-dissolution of impurities, which would again have a negative impact on the following steps. For large amounts of pDNA it has been suggested to multiply some devices (e.g. run them in parallel), which is not desirable and a disadvantage for a manufacturing scale.

Some centrifugation techniques could be run (semi-)continuously, but due to the sensitivity of polynucleotides to shear forces this treatment may cause degradation of plasmid DNA and genomic DNA and also detachment of precipitated impurities by rupture of the flocs. In case a conditioning step is applied before final purification, the salt composition and/or the conductivity and/or the pH-value of the cleared lysate has to be adjusted to a predetermined value that ensures binding of the desired molecule to the resin in the subsequent capture step. Sometimes the conditioning step is added for reasons of pre-purification (e.g. removal of endotoxins as described in WO 00/09680).

For capturing the polynucleotide of interest, several techniques are well known in the art, e.g. tangential flow filtration (WO 01/07599), size exclusion chromatography (WO 96/21729, WO 98/11208), anion exchange chromatography (WO 00/09680, US 6,410,274, WO 99/16869) and hydrophobic interaction chromatography (WO 02/04027).

Most of the described process steps are operated in a non-continuous and/or non-automated mode. Commonly the procedural steps are not connected to a fully continuous system.

In EP 0814156, WO 93/11218, EP 0616638 and EP 0875271 processes are disclosed wherein cell lysis, neutralization, clarification, washing, optionally conditioning and capturing are carried out in the same apparatus. These methods are open systems that are operated in a non-automated/non-continuous mode including several holding steps. The devices are only suitable for laboratory scales and cannot be transferred to manufacturing scales. The techniques also lack reproducibility and suitability for cGMP large-scale production.

Furthermore, combinations utilizing different devices have been described, wherein the individual steps are directly connected to each other in a continuous mode (WO 96/02658, WO 00/09680, WO 02/26966, US 2001/0034435 WO 97/23601, WO 00/05358, WO 99/37750). But none of these processes combines more than three steps within the series starting with a re-suspension step and ending with a capture step. The devices used in these methods do either not guarantee homogenous mixing or may apply disadvantageous shear forces to the solutes. Furthermore, the applied clarification techniques (filtration, centrifugation) are hampered by several drawbacks described previously.

A method and devices allowing the combination of more than three steps in an automated continuous mode suitable for the industrial production of a polynucleotide of interest is disclosed in WO 2004/085643 and by Urthaler J, Ascher C, Wöhrer H, Necina R (2007) (J Biotechnol 128:132). According to this process isolation of a polynucleotide which is not secreted by the host cell includes an improved alkaline lysis method as the cell disintegration step, a neutralization step, a clarification step, and optionally a conditioning step and/or a concentration step followed by the purification of the biomolecule. The clarification is carried out by allowing the mixture which comprises the precipitate and the lysate obtained during neutralization, to gently distribute and separate in a clarification reactor which is in its lower section partially filled with retention material. The precipitate is retained on the top of and within the layer of the retention material. The cleared lysate is continuously gathered via an outlet in the bottom of the reactor. Although this method and this devices are scalable and work in a continuous mode, the capacity of the clarification device is limiting. To increase the amount of biomass that can be processed within this setup the volume of the clarification tank has to be increased or another clarification tank must be applied to collect the whole floc-volume, consequently increasing equipment costs. Alternatively the production process has to be discontinued to remove the flocs from the clarification reactor, thereby delaying the production process and causing additional working-effort and furthermore increasing the risks for contamination.

### Summary of the invention

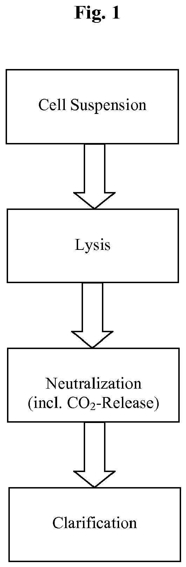

It was an object of the invention to provide a method and a device for isolating polynucleotides of interest from a cell culture to overcome the limitations of the known methods. Such method and device should be also suitable for a continuous production of therapeutically applicable polynucleotides. Thus, such process should neither require the use of enzymes like RNase and lysozyme nor the use of detergents apart from SDS. Furthermore the method and device should be suitable for industrial scale production of large amounts of the polynucleotides up to kilograms. A prerequisite for an industrial-scale production process is a completely continuous performance of cell lysis, neutralization and the subsequent clarification process (see Fig. 1).

To solve the problem underlying the invention, the following steps were taken:
Based on the method and device disclosed in WO 2004/085643 a number of experiments were carried out to overcome the limitation of the clarification step described in WO 2004/085643. Although the method and devices described in WO 2004/085643 are scalable and work in a continuous mode, the capacity of the clarification device is limiting.

These experiments were directed to improved floatation of the flocs and concurrently completely continuous separation of the flocs and the lysate. Beside aeration of the lysate-floc mixture different additives to the standard composition of the buffers were tested for improving floatation.

It was surprisingly found that addition of a carbonate salt prior, during or after the neutralization step led to a significantly improved floatation of the flocs generated during the neutralization step. Enhanced floatation is thereby mediated by small gas bubbles generated by a chemical reaction. When the carbonate as a solid salt or solubilized in a neutral to alkaline aqueous liquid comes into contact with an acidic solution CO₂ is released from the carbonate salt according to the following reaction equation:

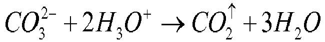

In one embodiment of the invention the carbonate salt is solubilized in a separate buffer/solution. In another embodiment the carbonate salt is solubilized in the resuspension buffer or in the lysis solution.

Alternatively the carbonate salt is solubilized in the suspension generated prior to the neutralization step (e.g. the carbonate salt is added to the buffer for resuspension or is added to the lysis solution). The concentration of the carbonate salt when applied solubilized with the resuspension buffer or the lysis solution (solubilized prior to neutralization) is in a range of about 0.01 to 1 M, preferably 0.02 to 0.1 M. Generation of the CO₂ bubbles starts when the lysed cell solution is contacted with the acidic neutralization solution and proceeds during mixing. The small gas bubbles attach to the concurrently generated precipitate (flocs of (potassium)dodecyl-sulfate co-precipitated with cell debris, proteins and genomic DNA) and thus promote the subsequent floatation. This process results in an excellent phase separation of the flocs and the lysate. This is the prerequisite for a complete continuous operation of this process step, for fast separation of flocs and clarified lysate and for a short contact time of the precipitated impurities and the pDNA-containing solution.

These results surprisingly revealed that a simple container with an inlet at a given position allows continuous exit (collection) of the (pre-)clarified lysate at the bottom of the container. The flocs, containing minimal residual lysate, could be collected at the top of the container. The container, which is also a subject of the present invention, is a How-through container, preferably a hollow body formed as a cylinder or tube, in particular a glass or stainless steel tube. The tube may also be made of plastic or any other material acceptable for biopharmaceutical production.

The collected flocs may be further processed e.g. for recovery of the residual inter-floc lysate by washing and/or draining. Beside conventional methods like centrifugation and/or filtration, in a preferred embodiment the clarification device described in WO 2004/085643 may be applied for these purposes. The lysate collected at the bottom outlet of the cylinder is further processed by subsequent purification steps.

The additionally recovered fluid from the floc washing and draining steps may be added to the lysate collected at the bottom of the cylinder. In an alternative embodiment the flocs collected at the top of the cylinder may be reprocessed by adding these flocs either to the neutralized lysed cell solution or directly to the mixture in the separation cylinder.

The continuous CO₂-release from carbonate salts (added in solubilized or solid form prior, during or after neutralization) under acidic conditions developed by a chemical reaction with the neutralized lysed cell solution in a continuous industrial scale alkaline lysis process resulting in improved continuous floatation of precipitate flocs generated during neutralization is novel. Furthermore the application/combination of this novel technique with the device allowing continuous separation of flocs and lysate at an industrial scale is novel. The combination of the novel CO₂-mediated floatation and the novel device for continuous floc separation with the automated continuous lysis and neutralization as described in WO 2004/085643 provides following outstanding advantages and solves the limitations of scale and is therefore crucial for economic production of large quantities of plasmid DNA:
- completely continuous and automated production chain for the generation of clarified lysate from biomass
- completely closed CIPable systems
- reduction of equipment size and costs
- improved floatation without additional devices or processing (e.g. without air injection)
- simple and free of complicated adjustments and maintenance demands
- robust and reproducible
- gentle (reduced mechanical stress on flocs resulting in reduced redissolution of precipitated impurities (and impurities attached to precipitate)
- increased yield due to a more compact floc layer and consequently less pDNA containing inter-floc-lysate)
- minimal contact time of precipitated impurities (flocs) and pDNA-containing liquid (lysate)

### Detailed description of the invention:

The present invention uses the release of CO₂ from carbonate salts under acidic conditions with the effect of improved floatation of a floccose precipitate generated during neutralization in a pDNA alkaline lysis procedure and to allow continuous separation of these flocs in an especially designed device. Thereby it is unessential when or to which buffer, solution or suspension the carbonate salt is added to the process as long as it is done prior to floc separation. Either the carbonate salt is added prior to neutralization under neutral or alkaline conditions or it is added to the already neutralized, floc containing lysed cell solution with an acidic pH. In all cases the CO₂ release takes place by contacting either a solution or a suspension containing the carbonate salt or the solid carbonate salt with the neutralized lysed cell solution or the neutralization solution.

The individual steps of the production process for biomolecules (especially pDNA) applying alkaline lysis are described below. Steps a) to c) and step e) may be performed according to known methods.

### a) Fermenting/cultivating (and optionally harvesting and resuspending):

In the method of the invention, preferably *E.coli* is used as host, in particular when the biomolecule of interest is pDNA. Fermentation is performed according to methods known in the art in a batch, a fed-batch or a continuous mode.

Harvesting is also performed according to methods known in the art. In one embodiment of the invention continuously operated devices, e.g. tube centrifuges or separators, are used for separating the cells from the cultivation medium. If the cells (the biomass) are frozen prior to further processing, the cells can be frozen (including cryopelletation) directly after harvesting or after resuspension of the cells in a suitable buffer, typically a buffer containing 0.05 M Tris, 0.01 M EDTA at pH 8. In this case no additional resuspension buffer has to be added prior to alkaline lysis or it is required in lower volume. In a preferred embodiment of the invention the resuspension buffer additionally contains the carbonate salt.

In an alternative embodiment of the invention, harvesting and resuspending the cells may be omitted. In this case the fermentation broth can be directly further processed in the lysis step b) without separation of cells and cultivation supernatant.

### b) Disintegrating by alkaline lysis:

In principle, step b) can be performed according to methods known per se, preferably according to methods that are gentle and can be run in a continuous and automated mode using an alkaline lysis solution that contains a detergent. A typical lysis solution consists of NaOH (0.2 M) and sodium dodecyl sulfate (SDS) (1%) (preferred), but in principle also other alkaline solutions, detergents and concentrations can be used (see e.g. WO 97/29190), in case of the method of the invention as long as a floccose precipitate is generated during the process. In one embodiment of the invention the lysis solution additionally contains the carbonate salt.

The harvested cells of step a) are either directly processed or thawn, if frozen before (e.g. including cryopelletation). Common to both procedures is that the harvested cells are resuspended in a resuspension buffer as described in a) prior to the intrinsic cell disintegration step b).

Alternatively the fermentation broth obtained in step a) is directly further processed without harvest and resuspension of the cells. In this case, the cells may be disintegrated by directly conducting alkaline lysis (and optionally subsequent neutralization) in a fermentor or by transferring the fermentation broth into the lysis reactor. In this embodiment without resuspension the carbonate salt may be added either to a neutral or alkaline fermentation broth, to the lysis solution or to the neutralized lysed cell solution to obtain the floatation improving effect of the CO₂ release (see step c)).

Preferably step b) is accomplished using the principle of the method and device described in WO 2004/085643.

In the following, with respect to cell disintegration in step b), the term "cell suspension" is used for both, the resuspended cells after harvesting and the fermentation broth.

### c) Neutralizing/precipitating/CO₂ release:

Typically a buffered solution with acidic pH and high salt concentration is used for neutralization. Preferably this solution contains 3 M potassium acetate (KAc) at pH 5.5. But also other neutralizing salts e.g. sodium acetate, ammonium acetate or potassium phosphate may be used or added.

Also this step may, in principle, be performed according to methods known per se, preferably according to methods that are gentle and could be run in a continuous and automated mode.

In one embodiment of the neutralization step c) the lysed cell solution is mixed with a neutralizing solution in a continuous, preferably automated manner. This can be accomplished by combining the lysed cell solution and the neutralizing solution e.g. by means of a T connector or Y connector, at a constant ratio of the flow rates (= constant mixing ratio) and ensuring mixing and thereby thoroughly neutralizing/precipitating of the solution in a subsequent mixing section.

In one embodiment of the invention, where the carbonate salt is added prior neutralization, the CO₂ release takes place concurrently to neutralizing/precipitating.

Preferably step c) is carried out using the principle of the method and device disclosed in WO 2004/085643.

Once the lysed cell solution is contacted with the neutralization solution, the pH of the mixture decreases to acidic and formation of the flocs starts. If the carbonate salt has been added prior to neutralization the CO₂ release and floc formation starts at the same time due to acidification. The lysed cell solution and the neutralization solution are then further mixed in a mixing section (e.g. in a tubing system as described in WO 2004/085643) and concurrently transported to the clarification device, preferably by pumps or pressurized gas.

In another embodiment, an aqueous "floatation solution" is applied. In the present invention the term "floatation solution" means an aqueous carbonate solution. This floatation solution is distinct from the other process solutions in that it contains the carbonate salt and is added to any of the other process solutions (The term "process solution" refers to any liquid that is used to perform alkaline lysis, neutralization and optionally resuspension). This floatation solution may be mixed with one of the process solutions prior the neutralization step or with one of the suspensions generated in the process. Alternatively the floatation solution may be mixed with the neutralized lysed cell solution. The concentration of the carbonate in the floatation solution is in the range of 0.01 M to 1 M, preferably 0.025 to 1 M. The mixing ratio of the floatation solution is thereby 2:1 - 1:50. Preferably higher concentrated carbonate solutions at a low volume are applied. When the floatation solution is applied continuously the mixing ratio is adjusted by the ratio of the flow rates (floatation solution : process suspension).

In this embodiment of the invention using the "floatation solution" in step c) the neutralized lysed cell solution may be mixed with the floatation solution in a continuous, preferably automated manner. This is accomplished by combining the neutralized lysed cell solution (containing the floccose precipitate) and the "floatation solution", at a constant ratio of the flow rates (= constant mixing ratio; e.g. by means of a T connector or Y connector) and ensuring thoroughly mixing and thereby CO₂ release during transportation of the reaction mixture to the subsequent clarification device. In this embodiment the "floatation solution" may be combined with the neutralized lysed cell solution at any point between the meeting point of the lysed cell solution and the neutralization solution and the clarification device, preferably within the first half of the distance.

Independent of concurrent (simultaneous to neutralization/precipitation) or subsequent (using the "floatation solution") CO₂ release the generated small gas bubbles get attached to the floc-precipitate, thereby improving phase separation of lysate and flocs during the later clarification. The calculated theoretical carbonate concentration in the resulting lysate-floc mixture (with attached CO₂ bubbles) should be in the range of about 0.003 to 0.35 M, preferably of about 0.005 to 0.05 M. Lower concentrations would result in negligible CO₂ release while too high concentrations would result in disadvantageous foaming.

For accomplishing the CO₂ release by one of the preferred embodiments a neutralization device as described in WO 2004/085643 is preferably used. This device is a tubing of about 3 - 200 mm inner diameter (depending on the scale of the process) preferably greater than 8 mm in order to avoid shear of the flocs at the tubing wall. The orientation of the flow may be in any direction, preferably upwards (form of a spiral). A mixing distance of 30 cm to several meters allows gentle and complete mixing of the solutions and thus precipitating the cell-derived impurities and CO₂ release. The mixing distance, the inner diameter of the tube as well as the retention time in the mixing device effect the quality of mixing and therefore the formation of the precipitate and CO₂ release.

In another embodiment including step d) in a non-continuous mode the carbonate salt is added as "floatation solution" or as solid salt to the neutralized lysed cell solution collected in the clarification device. The clarification device is preferably designed as described in WO 2004/085643. In this embodiment the "floatation solution" is preferably added from the bottom outlet in order to support homogeneous distribution of the floatation solution over the whole diameter of the clarification device via the retention material located at the bottom of the clarification device. For the embodiment with solid carbonate salt addition, the solid salt is added from an additional inlet at the top and homogeneous mixing may be achieved by mixers additionally installed in the lower part of the clarification device. Any other location or mixing method for the addition of the "floatation solution" or the solid carbonate providing sufficiently homogeneous CO₂ release in the neutralized lysed cell solution is possible to perform the improved gentle precipitate floatation of the method of the invention.

In all embodiments when a "floatation solution" (added after addition of the neutralization solution) is used, the mixing ratio (volume of added "floatation solution" per volume of neutralized lysed cell solution) is preferably chosen on the one hand to provide CO₂ release sufficient for complete and compact floatation of the precipitate and on the other hand to avoid too strong dilution of the lysate.

### d) Separating/clarifying (and optionally washing)

For the advantageous application of the method of enhanced floatation of precipitates generated during neutralization in an alkaline lysis process by CO₂ release from carbonate salt in an industrial scale process the method and device for the clarification (separation of the floccose precipitate) is crucial.

Three clarification modes for advantageous application of the method of the invention are possible:
I. continuous
II. semi-continuous
III. non-continuous (batch)

While the first clarification mode is carried out according to a method utilizing a device which are per se novel and a crucial part of the present invention, the second and the third mode of clarification are based in parts on the clarification-device and on the method already disclosed in WO 2004/085643.

In a continuous system (I) the CO₂ release usually takes place prior to the entry of the neutralized lysed cell solution into the clarification device. Nevertheless CO₂ release in the clarification device (as described in step c)) by addition of a floatation solution above the outlet of the clarification device described below is also possible. In a fully continuous system flocs and lysate are continuously separated in the clarification device. The lysate may be recovered at the bottom outlet and the flocs through an outlet at the top of the clarification device. An advantage of the method of the invention is that due to the continuous and thus fast separation of flocs and lysate the contact time is minimized, thereby minimizing the risk of degradation and introduction of impurities, which results in a high pDNA-quality in the collected lysate.

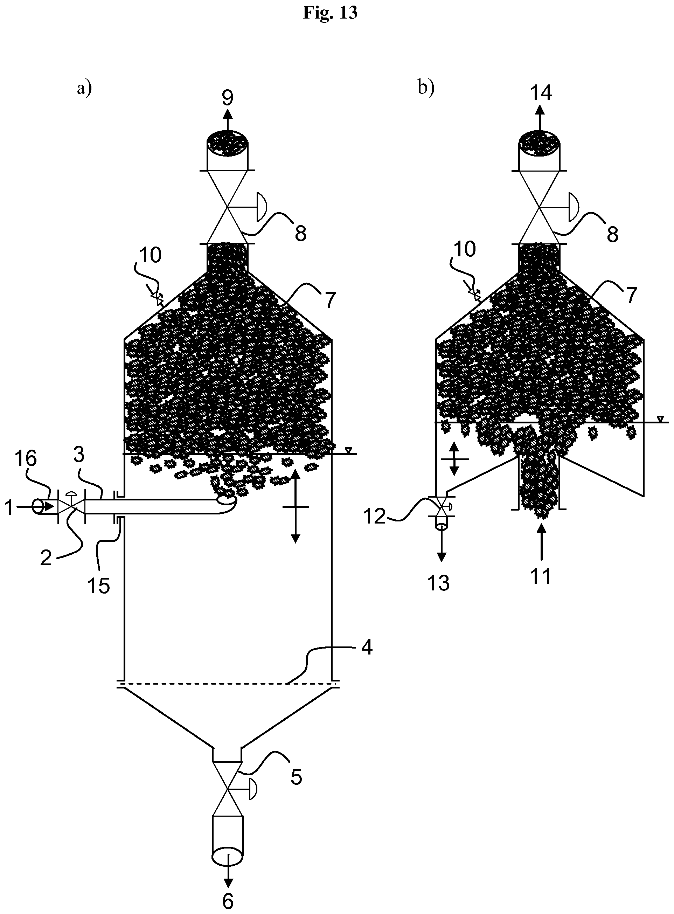

The novel clarification device can be made of glass, stainless steel, plastic or any other material that is acceptable for pharmaceutical production. The basic setup of the clarification device is shown in Figure 13a. The clarification device could also be extended, as shown in Figure 13b. The shape of the main part of the clarification device may be cylindrical, but in principle every other hollow body is applicable. Step d) in the method of the invention is independent of the shape of the reactor.

The clarification device has an outlet at the bottom (6) and another outlet at the top (9) which are advantageously designed to continuously recover pre-clarified lysate at the bottom and continuously remove floating precipitate flocs at the top. The outlets are preferably located cross sectionally central on top and bottom of the clarification device, although any other position on the bottom and top are possible. Furthermore the clarification device contains a port (15) at a position between the bottom and the top outlet, preferably in the middle of this distance. The outlets and the inlet (1) are preferably equipped with valves (2, 5, 8), which can be opened and closed separately. In the meaning of the present invention valves are any devices (preferably membrane valves) suitable to open and close conduits.

Since the novel clarification device can be operated in a fully continuous mode its size may be reduced compared to devices for semi-continuous or batch clarification. By example based on a predominantly cylindrical shape typical dimensions for industrial scale production, processing 10 - 1500 kg of biomass, preferably 50 - 750 kg, are 20 - 100 cm in diameter and 50 - 300 cm in length, preferably 30 - 80 cm in diameter and 100 - 250 cm in length. The diameter to length ratio should be in the range of 1:1 to 1:10, preferably 1:2 to 1:5. Increasing the length of the device may result in even better separation and drainage of the floating flocs.

The clarification device is equipped with a port (15) connected with the tubing in which neutralization/precipitation and preferably CO₂ release takes place. This port may itself be the inlet of the clarification device and is directly connected with the neutralization/ precipitation tubing. Alternative the port is connected with a distributing tubing (3) which itself is connected with the neutralization/precipitation tubing. In the embodiment wherein the port is the inlet, this inlet is a simple hole in the lateral jacket of the clarification device without any further parts projecting into the clarification device. In the other embodiment a preferably removable distributing tubing (optionally made of rigid material suitable for pharmaceutical production; e.g. stainless steel) extends into the clarification device. The tubing ends optionally in the middle in the clarification device. The end is open.

In both embodiments the port (connecting port in the first embodiment or end of extended tubing) is located as previously mentioned at a height between the bottom and the top outlet, preferably in the middle of this distance. The port/inlet may be of same or larger diameter compared to the neutralization tubing (16). In the second embodiment the port may be located at any position at the clarification device. Preferably the distributing tubing is oriented in a way that it allows upwards flow of the neutralized lysed cell solution and it ends preferably in the middle of the clarification device.

When the neutralized lysed cell solution, already containing flocs with attached gas bubbles, enters (1) the clarification device via the port (15) or the distributing tubing (3) immediately the phases separate. Due to the attached gas bubbles the density (mass per volume) of the flocs is significantly reduced compared to the process without CO₂ release. Therefore the flocs (7) start to float on the interface to the more or less clear liquid (lysate) in the clarification device. When the liquid level in the clarification device is above the inlet, the gas bubble-precipitate-complexes (7) are forced in upward direction to the top outlet.

At the beginning of the clarification step the clarification device is empty. The bottom outlet/valve (5/6) is closed till the clarification device is filled with neutralized lysed cell solution. Due to the attached gas bubbles the density (mass per volume) of the flocs is significantly reduced compared to the process without CO₂ release. Therefore the flocs (7) accumulate in the upper part of the device and the more or less clear liquid lysate in the lower part of the device.

Alternative, the clarification device is already filled with liquid, usually with a buffer containing components similar to the lysate. Starting the process, the bottom outlet/valve (5/6) is opened and the outflow adjusted in a way to maintain a constant level of the interface lysate-flocs in the clarification device throughout the process. The constant level of the interface lysate-flocs is controlled through the opening extent of the bottom valve, which optionally is automated. The volumetric outflow per time at the bottom is lower than the volumetric feed per time (inlet). Therefore the additional volume has to exit the clarification device via the top outlet (8/9). Since the interface lysate-flocs is below the top outlet predominantly the flocs (with minimal residual liquid between) are forced through the top outlet during the process. For ending of the process, the feed is be stopped by closing the inlet valve and the residual lysate is recovered through the bottom outlet (5/6) till the residual flocs floating on top of the lysate reach the bottom outlet (5/6).

Another option to the end the process is first closing the bottom outlet (5/6) and continue to feed the clarification device, optionally with a buffer solution containing components similar to the lysate, till all residual flocs are forced out of the device via the top outlet (8/9). The clear lysate present in the device is then simply recovered via the bottom outlet (5/6) as described above (top outlet (8/9) and inlet (1/2) closed; separate venting valve (10) at the top opened).

The two options for ending of the process could also be combined in the following way: First the lysate is recovered according to option 1 and second the flocs are forced out of the clarification device via the top outlet according to option 2.

In the alternative embodiment wherein CO₂ is released in the clarification device (as described in step c)) a "floatation solution" is added continuously to the lysate-flocs mixture in the clarification device via an additional port/inlet, designed similar to the one described above for the neutralized lysed cell solution. Optionally this port/inlet may be equipped with means for better flow distribution (e.g. a perforated plate or a frit) at the end connected with or reaching into the clarification device. This inlet has to be positioned between the bottom outlet and the inlet for the neutralized lysed cell solution.

The bottom part of the novel clarification device is optionally slightly conical in order to guarantee complete lysate recovery (complete discharge) and complete removal of cleaning solutions after stopping the process. This bottom part may be equipped with a fixture (4) suitable to retain residual non-floating flocs above the bottom outlet. For removable fixtures the bottom part of the clarification device is detachable from the residual device. Fixtures may be perforated plates, sieves, nets, frits or any other installation or material permeable for the lysate. The material of these fixtures may be as for the whole clarification system stainless steel, glass, polypropylene or any other material suitable for pharmaceutical production. In a special embodiment the fixture is a retention layer with similar or identical parts as the retention layer described for the clarification reactor in WO 2004/085643.

Additionally depth filters may be installed at the bottom outlet line of the described clarification device to ensure safety regarding undesired breakthrough of minimal amounts of non-floating flocs. Alternatively the recovered lysate may be fed into the clarification reactor described in WO 2004/085643 for fine clarification.

The top part of the novel clarification device is optionally tapering, preferably conical tapering, to an inner diameter similar to the inner diameter of the top outlet valve (8) and the outlet tubing following next to the valve consisting of any material suitable for pharmaceutical production. The inner diameter of the upper tapered end of the top part of the clarification device (and consequently the top valve and the following tubing) is in the range of about. 3 - 200 mm (depending on the scale of the process) preferably greater than 8 mm in order to avoid shear of the flocs at the tubing wall. The tapering-angle of the top part of the clarification device is in the range of 10° - 80°, preferably 25°- 65°. The tapering creates a bottleneck at the top outlet, which provides enhanced drainage of the floating flocs (7) and reduces loss of lysate through exported flocs. This tapering top part of the clarification device may be optionally detachable (e.g. for separate cleaning).

In another embodiment an additional unit (Figure 13 b) for the separation of flocs and lysate is connected to the top outlet/valve (8/9) of the clarification device, preferably in straight line. This additional unit is characterized by a shape similar to the upper part of the clarification device (main part cylindrical). The length of this additional unit may be shorter compared to the main clarification device e.g. about 1/3 of the length of the main device. The inlet (11) of this additional unit is connected to the top outlet (9) of the main clarification device at same inner diameter. Next to this inlet (11) the diameter of the additional unit is increased (e.g. to a similar inner diameter as the main clarification device). In a preferred embodiment the inlet (11) of the additional unit is not the lowest point of this unit. In this alternative embodiment the bottom of this additional unit is either descending from the inlet to the jacket over the whole bottom area or at a certain point. An outlet (13) with a valve (12) may be located at the lowest part of the bottom. When the pre-drained flocs enter the enlarged part of this additional unit next to its inlet (11) a further phase separation process takes place. While the flocs are again floating (7) to the top of the additional device, residual lysate previously trapped between the flocs accumulates at the lower area and could be recovered at the outlet (13) through the valve (12), which is optionally automatically, periodically or continuously opened in a way that minimal flocs are exported together with the lysate. The top of this additional unit is designed similar to the top of the main clarification device with an outlet (14), a valve (8) and a separate venting valve (10), and optionally contains a second outlet. In the embodiment with a single top outlet (14) the flocs are exported in the same way as described for the main clarification device. In the embodiment with two top outlets one of the outlets is equipped with a removable frit or a connected filter, representing another point of lysate recovery. When the valve of the second top outlet is closed during the process (assumed that the bottom outlet (of this additional unit is closed) lysate is exported through the other top outlet while flocs are retained by the frit or the filter.

The lysate additionally recovered in this unit may be added to the clarified lysate recovered at the outlet of the main clarification device (6) or to the lower lysate phase in the main clarification device by appropriate tubings.

The main clarification device may be expanded (cascade like) with more than one of the above described additional units.

In yet another embodiment another additional clarification unit may be connected to the top outlet of the main clarification device. This additional unit consists of a tube put into another tube (tube-in-a-tube). The inner tube is connected to the top outlet of the main clarification device and has an inner diameter similar to this outlet or smaller but at minimum 8 mm. The outer tubing surrounds the inner tubing over its whole length and has an outlet (optionally equipped with a valve) next to the connection of the inner tubing to the main clarification device. The outer tubing is fixed to the inner tubing at both ends by e.g. suitable gaskets. The direction of this tube-in-a-tube combination is (slightly) inclined upwards from the outlet of the main clarification device. The length is in the range of 30 cm to 3 m, preferably in the range of 0.5 - 2 m. This inner tube is perforated over its whole length in the radially lower half of the jacket, preferably with (round) holes of a size (diameter 0.5-3 mm) avoiding passage of the flocs. The flocs exported through the outlet of the main clarification device are forced through the inner tube. Due to the attached gas bubbles the flocs are predominantly transported at the radially upper part of the tubing, while the residual lysate can exit the inner tubing through the perforation and is collected in the outer tubing and recovered at the outer tubing outlet. Thereby the flocs are further drained and lysate recovery increased. The lysate additionally recovered in this unit may be added to the clarified lysate recovered at the outlet of the main clarification device or to the lower lysate phase in the main clarification device by appropriate tubings.

In yet another embodiment the top part of the novel clarification device may be equipped with mechanical skimmers, which continuously skim the flocs exiting at the top outlet of the clarification device. In this embodiment the top outlet is equipped with a skimming top allowing collection of the skimmed flocs for further processing.

In yet another embodiment the flocs can simply overflow at the top outlet and be collected in a collection ring attached radially to the top outlet. By overflowing the top outlet the flocs may be drained and washed by flowing over a perforated screen (with a ring to collect drained lysate below) before falling into the collection ring. The bottom of the collection ring is preferably inclining directing the collected flocs automatically to a tank collecting the drained ("dry") precipitate.

In yet another embodiment the floating flocs may be sucked off at the top outlet of the main clarification device by means of pumps.

Every additional clarification unit described above may be combined with another one. The separated flocs are optionally further processed (e.g. residual draining, washing). Residual draining may be accomplished according to methods known in the art e.g. filtration (preferably depth filtration), application of filter presses, centrifugation or equivalents. An advantageous gentle draining method, which can be combined with a floc washing step is described in WO 2004/085643 utilizing an especially designed clarification reactor including a gentle distributor with a retention material in the bottom. The method and device described therein can be easily combined with the present invention by applying the exported flocs to the clarification reactor of WO 2004/085643 and performing the drainage and washing procedure described therein.

For the washing solution/buffer a composition is chosen that does not re-dissolve the flocs. Washing may also be carried out by combining the stream of exiting flocs and washing solution/buffer and mixing/contacting it in a device similar to the neutralization tubing (WO 2004/085643). Optionally the flocs may also be washed in a separate tank (batch or fed-batch mode).

In another embodiment of the invention the flocs may be recycled in the novel clarification device. Therefore a part of the floating flocs exported at the top outlet of the clarification device are transported (e.g. by pumps) back to the clarification device via suitable tubing and a separate inlet near the inlet of the feed solution. For this embodiment it is advantageous to supply additional floatation solution in the novel clarification device.

All valves in the described embodiments are preferably membrane valves but may also be e.g. ball valves, gate valves or anything else suitable to open/close lines/pipes and/or containers. Some of the valves described in the embodiments may also be omitted (e.g. top outlet valves) without influencing the basic/principle character of the invention.

In the semi-continuous system (II) the CO₂ release usually takes place prior to the entry of the neutralized lysed cell solution into the clarification device. Nevertheless CO₂ release in the clarification device (as mentioned in step c)) by adding of a floatation solution above the outlet of the clarification device described is also feasible.

For the application of the method of the invention in a semi-continuous mode the clarification reactor as disclosed in WO 2004/085643 is preferably used as clarification device. The neutralized lysed cell solution (containing the precipitate) may be applied via a top inlet and the original or adapted special designed distributor described in WO 2004/085643.

In another embodiment the inlet may be at any position of the clarification reactor which is above the retention material. Due to the improved floatation by the method of the present invention based on the CO₂ release from carbonate salt by acidification, the risk of blockage of the retention material during the process is significantly reduced. Therefore the thickness and composition of the retention layer may be adapted e.g. reduction of the number of layers or change of the fineness/porosity of the retention material. By example only course glass beads may be used above the bottom frit.

In another embodiment of the semi-continuous mode of the method of the invention only minimal (e.g. a single use frit) or no retention material is utilized in the clarification reactor of WO 2004/085643. Additionally, depth filters may be installed at the bottom outlet line of the clarification reactor to ensure safety regarding undesired breakthrough of minimal amounts of non-floating flocs.

The semi-continuous process ends when the clarification reactor is completely filled with (floating) flocs. The flocs in the clarification reactor may be washed and drained according to methods described in WO 2004/085643.

Although the clarification reactor described in WO 2004/085643 is beneficial and preferred for carrying out the method of the invention in a semi-continuous mode any other hollow body with an inlet and a bottom outlet for (semi-)continuous recovery of the lysate is suitable for this purpose.

An advantage of the semi-continuous clarification mode compared to other semi-continuous clarification methods known in the art is its increased capacity. Due to the improved floatation by CO₂ bubbles attached to the precipitate flocs the layer of floating flocs is more compact trapping less lysate between the flocs. Therefore more biomass can be processed with a given volume for accumulation of the flocs in the clarification device. The recovery of separated lysate phase is much easier than recovery of lysate trapped within the flocs.

The system/method for semi-continuous clarification as described above may also be applied/performed in a non-continuous (batch) mode.

In a non-continuous (batch) system (III) the CO₂ release takes place in the clarification device after the entire neutralized lysed cell solution is filled in the device, leaving some space for the addition of a floatation solution. CO₂ release is carried out directly in the clarification device (as mentioned in step c)) by addition of a floatation solution above the outlet of the clarification device. This non-continuous system is limited by the volumetric capacity of the clarification device. Once the clarification device is almost completely filled with neutralized lysed cell solution, containing the floc precipitate, CO₂ -mediated floatation is started. The bottom outlet is closed till floatation is finished. Afterwards the lysate is recovered in a similar way as described for the semi-continuous system. The clarification device may be designed similar to examples given for the semi-continuous system, preferably according to the clarification reactor as described in WO 2004/085643 (similar adaptations/changes as described above for the semi-continuous system possible). Optionally draining and washing of the flocs is carried out as described for the semi-continuous system.

The resulting lysate recovered by anyone of the systems described above is either optically clear and can directly be further processed e.g. captured, usually by chromatographic techniques or is further purified by additional simple fine clarification e.g. filtration.

### e) Purifying:

A process following steps a) to d) of the invention facilitates isolating (capturing) and purifying of the biomolecule of interest in the subsequent steps (e.g. continuous or non-continuous concentration, conditioning, filtration, chromatography).

The process of the invention with improved floatation utilizing one of the clarification systems described above is suited for, but not limited to, biomolecules that are sensitive to shear forces, preferably to polynucleotides, in particular plasmid DNA, and large proteins, e.g. antibodies.

The process of the invention can be used for any biomolecule of interest. For the production of proteins, it may be designed such that the specific needs of the protein of interest are met. The method of the invention is independent of the fermentation process and of the source of the protein (e.g. bacteria, yeast).

The choice of specific methods suitable for cell disintegration and the following processing steps are strongly influenced by the protein's state in the cells after fermentation:
If the protein is over expressed, it may be present in the form of so-called "inclusion bodies". In this case, the treatment with e.g. strong alkali in combination with a reducing agent (e.g. dithiothreitol, DTT) during lysis results in a resolubilization of the protein, which is, at this stage, in its denatured form. To reconstitute the protein's native structure, refolding can be achieved by neutralization (e.g. by addition of phosphoric acid), concurrent to the CO₂ release, in the neutralization reactor or in a second reactor similar to the lysis reactor. Insoluble components are separated from the protein-containing solution in the clarification device.

In the case the protein of interest is soluble in the cell, the cells are disintegrated in the lysis reactor in a similar manner as described above .

In the lysis reactor, the conditions (contact time, concentration of the lysis solution) may be chosen in a way that the protein stays soluble or, alternatively, the parameters are set to specifically denature and precipitate the protein. In the first case, the solution is further processed in the neutralization reactor (which, in terms of construction, is similar to the lysis reactor or the neutralization reactor used for polynucleotides) and the clarification device, as described for solubilized inclusion bodies. If the protein is in its denatured state, precipitation can either already take place in the lysis reactor or afterwards in the neutralization reactor (by addition of a neutralizing and/or precipitating agent concurrent to CO₂ release). In both cases, the conditions for the precipitation are preferably chosen to specifically precipitate the protein of interest (while undesired impurities like e.g. RNA, endotoxins, and DNA stay soluble). The precipitate is subsequently separated from the solution in the clarification device. Afterwards, the precipitate is either removed from the clarification device (continuously as described for the continuous system (I) of the present invention or e.g. by sucking off or flushing out with an appropriate buffer) or directly further processed in this device. After it has been removed from the device, the precipitate (protein of interest) is resolubilized in a separate container using a suitable buffer, which is empirically determined on a case-by-case basis. In the case the precipitate remains in the clarification device, resolubilization is performed there (by addition of a suitable buffer and optionally mixing). As soon as the precipitate (especially the protein of interest) is resolubilized, it can be easily removed from the clarification device through the outlet in the bottom.

Common to all variations of the method of the invention in the production of proteins are the options for further processing the resulting protein solution. Beside additional refolding steps, the same steps as described for processing of polynucleotide solutions (continuous or non-continuous concentration, conditioning, filtration, capturing) may take place.

The process of the invention meets all regulatory requirements for the production of therapeutic biomolecules. When applied to polynucleotides, the method of the invention yields - provided the fermentation step has been optimized to provide high quality raw material - high proportion of plasmid DNA in the ccc form and a low proportion of impurities (e.g. proteins, RNA, chromosomal DNA, endotoxins). The process neither requires the use of enzymes like RNase and lysozyme nor the use of detergents except in lysis step b). The contact time of lysate and floccose precipitate before clarification is significantly reduced. Furthermore the process is carried out without supplying gas (from an external source).

The process of the invention is scalable for processing large amounts of polynucleotide containing cells, it may be operated on an "industrial scale", to typically process more than 1 kilogram wet cells, and yielding amounts from 1 g to several 100 g up to kg of the polynucleotide of interest that meet the demands for clinical trials as well as for market supply.

The applicability of the process is not limited or restricted with regard to size, sequence or the function of the biomolecule of interest. A polynucleotide of interest may be a DNA or RNA molecule with a size ranging from 0.1 to approximately 100 kb or higher. Preferably, the polynucleotide of interest is circular DNA, i.e. plasmid DNA with a size of preferably 1 to 20 kbp (without limitation).

The process and the devices of the invention are not limited with regard to the cell source from which a biomolecule of interest is to be obtained.

The process can be easily implemented and is flexible with regard to automation and desired scale; adjustment of the flows and the reaction times can be achieved by commercially available pumps and pressure systems that ensure steady flows and a low impact of mechanical stress.

Another advantage of the present invention is that the devices are sanitizeable, depyrogenizeable and allow cleaning in place (CIP) and steaming in place (SIP).

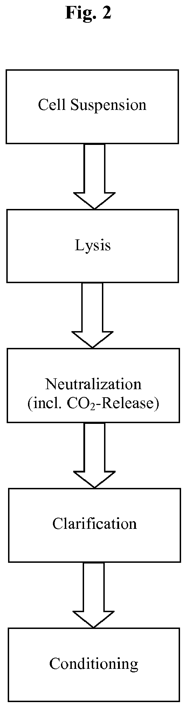

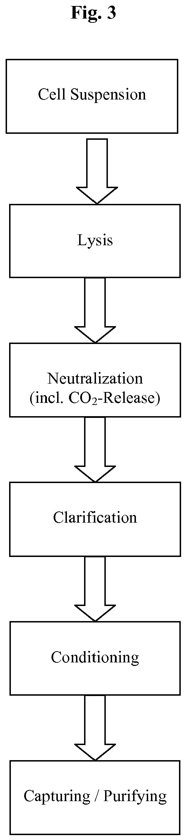

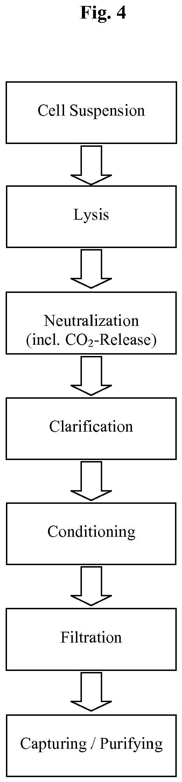

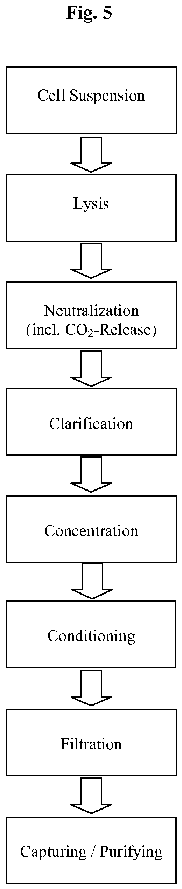

The method and apparatus employed therein provides a controllable and consistent performance in a closed system, allowing direct further processing of the continuously produced lysate obtained after clarification, e.g. loading it to a chromatography column or allowing online conditioning and/or filtration of the lysate prior to column loading (Fig. 2-4). After clarification, there may be an intermediate concentration step before conditioning or loading onto the chromatographic column (Fig. 5).

In the process of the present invention, irrespective of whether step a) is performed batchwise or in a continuous mode, each subsequent step may be run in a continuous and automated mode. Preferably, a combination of at least two steps selected from steps b) to e) is run continuously connecting the individual steps.

In the case the lysis step b) is the automated/continuous step, it is independent of how the cell suspension has been obtained (batchwise or continuous operation, direct use of fermentation broth or harvest and resuspension, optionally after freezing). It is also independent of the host from which the lysate has been obtained.

In the case the neutralization step c) is the automated/continuous step, the application is independent of how the processed alkaline lysed cell solution has been prepared (e.g. batchwise or continuous). In a preferred embodiment the collector tank following the neutralization step is designed in the same way as the clarification reactor described in WO 2004/085643 (in the case clarification is carried out batchwise or semi-continuous). In the case the clarification step d) is the automated/continuous step, the application is independent of how the processed neutralized lysed cell solution containing flocs has been prepared (e.g. batchwise or continuous). It is also independent of when and where (prior to the clarification device or in the clarification device) the CO₂ release of the method of the invention takes place as long as it is prior (or during) the clarification process. It is furthermore independent of how the resulting clarified lysate is further processed.

In a preferred embodiment, the outflow of the clarification device is combined with the flow of the solution necessary for the next processing step (e.g. conditioning solution) by means of a connector, e.g. a T- or Y-connector or directly in a mixing device. The two solutions may be pumped by conventional pumps at certain flow-rates.

In another embodiment only the flow rate of the second solution is adjusted to the flowrate of the lysate leaving the clarification device. The mixing device for this purpose may be a device filled with beads like the one described for the automated lysis step or a tubing system like the one described for the neutralization step (WO 2004/085643). Such a setup may be used if conditioning of the lysate for the first chromatographic step is necessary. For example, a solution of ammonium sulfate (or simply water) can be added in this way. In another embodiment, the process also comprises an intermediate concentration step (Fig. 5): as soon as a sufficient volume of the lysate leaving the clarification device is present, the lysate is concentrated, e.g. by means of ultrafiltration, prior to conditioning and/or loading onto the chromatography column. Concentration may be performed in one or more passages and per se carried out in a continuous or batchwise mode. If only one passage takes place, the retentate (e.g. containing the pDNA) may subsequently be directly conditioned or loaded to a chromatography column. In the case of several passages, the retentate is recycled until the desired final volume/concentration is reached, and subsequently further processed. For this concentration step, conventional devices can be used, e.g. membranes in form of cassettes or hollow fibers. The cut-off of suitable membranes depends on the size of the biomolecule processed. For pDNA, usually membranes with a cut-off between 10 and 300 kDa are used.

In a preferred embodiment, the lysis reactor and the neutralization reactor are combined to form a two-step automated/continuous system. In this case, the outflow of the lysis reactor is connected and mixed with the flow of the neutralization solution in the manner described for the automated/continuous neutralization step (WO 2004/085643). By this, the flow rate of the pumped neutralization solution is adjusted to the flow rate of the outflow of the lysis reactor.

In another preferred embodiment the neutralization reactor and the clarification device are combined to form a two-step automated/continuous system. In this case, the outflow of the neutralization reactor is connected with the automated/continuous clarification device of the invention. In this case, the degree of opening of the bottom outlet valve (and optionally the top outlet valve) of the clarification device has to be adjusted such that the level of the interface (interface height in the clarification device) of floating flocs and clear lysate is kept constant. This may be achieved by measuring the interface level by means of an integrated floater, which floats on the liquid but not on the floes. Another option is measuring the flow at the bottom outlet of the clarification device, which can be used for the calculation of the theoretical level of the interface according to a special algorithm based on empirically defined parameters (distribution coefficient). The bottom outflow may never be less than 50 % of the feed flow. Also other systems like light barriers are applicable. In principle every system suitable to recognize the interface can be used. By means of an electronic connection to the outlet valve the outflow can be adjusted stepwise or stepless according to the floc-lysate interface level or the outlet flow.

In another embodiment the lysis step and the clarification step are connected by directly connecting the two devices without an intermediate distinct neutralization step.

Neutralization may in this case be carried out in the clarification device of the non-/semi-continuous system (system II and III). In this embodiment neutralization and clarification are therefore carried out non-continuously. The outlet of the non-continuous clarification device is closed at first and the lysed cell solution is combined with a certain volume of neutralization solution. The neutralization solution may be presented in the clarification device. If the neutralization solution is added after the whole lysed cell solution is collected in the clarification device this is preferably done via a bottom inlet in the non-/semi-continuous clarification device. In both cases mixing with the lysed cell solution may be enhanced by (slowly) stirring with a stirrer or introducing air through the inlet in the bottom of the device or an additional inlet below the fluid level. In this embodiment with direct connection of the lysis step with the clarification device the CO₂ release takes place in the clarification device. The carbonate(-salt) is thereby preferably a component of the lysed cell solution or is additionally added prior or after neutralization (as solid salt or as "floatation solution", which would preferably be added through the bottom outlet of the non-/semi-continuous clarification device (system II and III)). At the end of neutralization/floatation, non-continuous-clarification takes place in the same manner as described in WO 2004/085643.

In an even more preferred embodiment, the whole system is fully automated by employing at least all steps b) to d) and optionally, in addition, step a) and/or e) in a continuous system. In this embodiment, the outflow of the lysis reactor is directly connected with the neutralization device and the outflow of the neutralization device is directly connected with the clarification device. In this embodiment the fully continuous system (I) or the semi-continuous system (II) for improved clarification by CO₂ release would be applied. The design for the individual connections and devices is the same as described above.

In a most preferred embodiment, the fully automated system is connected to an optional automated (and continuous) conditioning step (and device). This embodiment allows continuous mixing of the clarified lysate that leaves the clarification device with a conditioning solution (e.g. an ammonium sulfate solution). As described above, such conditioning step may be necessary to prepare the polynucleotide containing lysate for the subsequent (chromatographic) purification steps (e.g. hydrophobic interaction chromatography).

Adding such a conditioning step results in an extension of the automated and continuous three-step system to a continuous four-step system. In this embodiment, a conditioning solution can be continuously mixed with the clarified lysate using a device, which is preferably of the same type as the lysis reactor. This device was found to be most gentle for continuous mixing of solutions containing polynucleotides that are sensitive to shear forces. Yet also other devices (e.g. as described for the neutralization step) can be utilized for this purpose, e.g. conventional static mixers. The flow rate of the pump that pumps the conditioning solution can be adjusted to the flow rate of the outflow of the clarification device by installing a flow measurement unit. The pump can be connected with this unit and thus regulated, keeping the ratio of the flow rates of the two mixed solutions constant. Between conditioning and capture step, an on-line filtration step may be inserted.

In yet another embodiment of the invention, an ultrafiltration step is added. By such an extension of the automated three-step system, the process represents a continuous four-step system. In this embodiment the resulting lysate of the previous steps is concentrated by ultrafiltration. While the permeate is discarded, the retentate is either directly further processed by the conditioning step and/or by the loading step (which means an extension of the continuous system by one or two additional steps) or recycled until a desired final concentration/volume is reached. In the latter case, the resulting concentrate is further processed (conditioning and/or loading) after concentration is finished.

In another embodiment, the lysate flowing out of the clarification device may be directly loaded onto a chromatographic column, or it may be loaded onto the column after concentration and/or conditioning (with or without subsequent on-line filtration).

In all described embodiments utilizing the automated improved (CO₂ release) clarification step the obtained cleared lysate may either be collected in a suitable tank or directly further processed (e.g. by connecting the outflow of the clarification device with another device, e.g. a chromatographic column). If a concentration and/or conditioning step is employed in this automated process, the concentrated and/or conditioned lysate can either be collected in suitable tanks or directly further processed.

The method and device of the invention are independent of the pumps used for pumping the solutions. In a special embodiment, the flow of the several suspensions and solutions is accomplished by air pressure in pressurized vessels instead of pumps.

The process and device of the invention are suitable for cGMP (Current Good Manufacturing Practice) production of pharmaceutical grade pDNA. The process can be adapted to any source of pDNA, e.g. to any bacterial cell source. In particular due to the properties of the system, the process of the invention allows fast processing of large volumes, which is of major importance for processing cell lysates. Since the lysate contains various pDNA-degrading substances such as DNases, process time is a key to high product quality and yield. In this context especially the short contact time of the floccose precipitate and the lysate before clarification, enabled by the method of the invention is of major advantage.

The process and device of the invention are suited for production of pDNA for use in humans and animals, e.g. for vaccination and gene therapy applications. Due to its high productivity, the process can be used for production of preclinical and clinical material as well as for market supply of a registered product.

Since the method and device of the invention enable completely continuous execution of the alkaline lysis, the neutralization and the clarification and corresponding and connected steps as described above, method and device of the invention are completely scalable (allowing processing of biomass obtained from fermentations up to 4000L or even more).

### Brief description of the drawings

Figure 1: Flowchart of a combined continuous three step process comprising alkaline lysis, neutralization (including concurrent/subsequent CO₂ release) and clarification.
Figure 2: Flowchart of the combined continuous three-step process of Figure 1, extended by a continuous conditioning step (e.g. concentration and/or high salt precipitation).
Figure 3: Flowchart of the combined continuous process of Figure 2, extended by an additional capture step.
Figure 4: Flowchart of the combined continuous process of Figure 3 including an on-line filtration step between conditioning and capture step.
Figure 5: Flowchart of the combined continuous process of Figure 4 extended by a concentration step before conditioning.
Figure 6: Scheme for the continuous combination of alkaline lysis reactor, neutralization reactor and the (adapted) semi-continuous clarification device of WO 2004/085643, applicable for clarification mode "system II" and "system III".
Figure 7: Clarification mode "system II": Comparison of floating flocs (in the adapted pilot-scale clarification device of WO 2004/085643) obtained by the novel method with improved floatation (a) and by the standard method (b) (without CO₂ release). The upper images show the complete floc layer while the lower images show a zoomed part of the floc layer.
Figure 8: Analytical HPLC chromatogram of a reference lysate obtained by a conventional manual method on the laboratory scale (without CO₂ release).
Figure 9: Clarification mode "system II": Analytical HPLC chromatogram of a lysate obtained by the continuous method of the invention including the steps lysis, neutralization (incl. CO₂ release) and semi-continuous clarification in the adapted up-scaled device of WO 2004/085643.
Figure 10: Analytical HPLC chromatogram of the SEC Pool as last purification step of a pDNA containing lysate obtained by the continuous method of the invention including the steps lysis, neutralization (incl. CO₂ release) and semi-continuous clarification in the adapted up-scaled device of WO 2004/085643 - clarification mode "system II".
Figure 11: Clarification mode "system II": Floating flocs in the adapted up-scaled clarification device of WO 2004/085643, obtained by the novel integrated floatation method of the invention.
Figure 12: Clarification mode "system II": Procedure of floc washing (flocs separated by the novel method of the invention) utilizing a CIP ball at the top of the adapted up-scaled clarification device of WO 2004/085643 at the end of the recovery process.
Figure 13: Clarification mode "system I": Scheme of the novel continuous clarification device of the invention - a) Basis setup, b) example for a preferred optional extension part of the basis setup.
Figure 14: Clarification mode "system I": Lab-scale development set-up
Figure 15: Analytical HPLC chromatogram of a reference lysate obtained by a conventional manual method on the laboratory scale (without CO₂ release).
Figure 16: Clarification mode "system I": Analytical HPLC chromatogram of a lysate obtained by the continuous method of the invention including the steps lysis, neutralization (incl. CO₂ release) and continuous clarification with the lab scale development set-up.
Figure 17: Analytical HPLC chromatogram of the SEC Pool as last purification step of a pDNA containing lysate obtained by the continuous method of the invention including the steps lysis, neutralization (incl. CO₂ release) and continuous clarification ("system I") in the lab scale development set-up.
Figure 18: Clarification mode "system I": Prototype lab-scale set-up

### Example 1

### Production of pDNA-containing E. coli cells

The pDNA containing *E. coli* biomass was produced according to WO 2004/085643 or according to WO2005/097990.

### Example 2

### Verification of the principle applicability of improved floatation by CO₂ release from carbonate(-salt) during neutralization in an alkaline lysis process

First experiments were carried out with buffers only, without biomass. Different carbonate-salts (e.g. K₂CO₃ and NaHCO₃, which are advantageous since K⁺ and Na⁺ as counter-ions to CO₃²⁻ are already present in the buffers/solutions used for lysis or neutralization) were added to the resuspension buffer or the lysis solution in a concentration of 0.5 M. While solubility in the buffers were examined on the one hand, on the other hand the intensity of CO₂ release during neutralization and consequences on floatation of flocs (generated during neutralization) were examined.

It was observed that 0.5 M NaHCO₃ could be well solubilized in the buffer usually used for resuspension of the biomass (containing 0.05 M Tris and 0.01 M EDTA at pH 8) and resulted in extensive CO₂ release when acidified by mixing with the neutralization solution generating floating foam of precipitated SDS.

This experiment was repeated with 5 g biomass, which were resuspended in 50 mL resuspension buffer. The concentration of NaHCO₃ added to the resuspension buffer was reduced to 0.1 M in order to avoid too strong foaming (as observed in the first experiment). The resuspended biomass was gently mixed with 50 mL lysis solution (0.2 M NaOH, 1 % SDS), contacted for 2 min and afterwards neutralized with neutralization solution (3 M KAc). This setup worked very well regarding moderate (not too extensive) CO₂ release and concurrently enhanced floatation of the precipitate-flocs by attached CO₂ bubbles. The lysate below the floating flocs was analyzed (HPLC) and compared with a lysate obtained by the standard procedure without CO₂ release.

The pDNA-concentration (yield) in the lysate obtained with CO₂ release was about 70 % of the reference lysate. The pDNA homogeneity was greater than 80 % in both cases. The layer of floating flocs was much more compact in the experiment with the CO₂ release (compared to the experiment without CO₂ release), which poses a major advantage for clarification on the industrial scale.

This initial experiment showed principle applicability of the carbonate salt based CO₂ release for improved floatation of precipitate flocs generated during the neutralization step of an alkaline lysis procedure for pDNA production.

### Example 3

### Optimization of the carbonate-concentration (in the resuspension buffer)

These experiments were carried out as described in Example 2 using different NaHCO₃ concentrations (0 M = reference, 0.05 M, 0.07 M, 0.1 M and 0.2 M) in the resuspension buffer (P1) and 1.1 g biomass respectively. After neutralization the floc containing lysate was hold for 3 min and afterwards clarified by centrifugation (lab centrifuge at 7500 rpm). The lysate (after centrifugation recovered as the supernatant) was analyzed regarding concentration (yield) and pDNA homogeneity (HPLC). The pellets were washed and the wash-fractions also analyzed in the same manner. Furthermore the impact of the addition of the NaHCO₃ on the pH of the resulting mixture with lysate solution (P2) was investigated. The experiments were carried out in 4-fold repetition. the results are summarized in Tab. 1.

**Tab. 1: Results (average of 4 repetitions) of Example 3**

| NaHCO₃ conc. in PI (M) | Yield (µg/mL/%) | Homogeneity (% ccc) | pH of P1/P2 mixture |
|---|---|---|---|
| 0 = Reference | 80.6/100 | 73.7 | 12.7 |
| 0.05M | 81.8/102 | 72.8 | 12.6 |
| 0.07M | 74.1/92 | 69.6 | 12.5 |
| 0.10M | 71.2/88 | 71.7 | 12.4 |
| 0.20M | 38.8/48 | 71.3 | 10.9 |

Optimal yield was obtained, when the resuspension buffer contained 0.05 M NaHCO₃. When higher concentration of the carbonate salt were used yield decreased and dropped significantly for 0.2 M. pDNA homogeneity was relatively stable and seems not to be significantly affected. The lower yield at the higher NaHCO₃ concentrations is mainly caused by the resulting lower pH of the mixture of resuspension buffer and lysis solution. Since the pH is critical for degree/completeness of cell disintegration the pH reducing effect of the carbonate salt has to be considered for higher concentrations. In the example described here the critical pH is at 12.5. Therefore higher NaHCO₃ concentrations should not be applied without changing the NaOH concentration in the lysis solution or its applied volume per volume resuspended biomass.

All analyzed wash fractions showed similar pDNA concentration and homogeneity.

### Example 4

### CO₂ release from carbonate(-salt) during neutralization and improved floatation in a process utilizing continuous alkaline lysis, neutralization (CO₂ release) and semi-continuous clarification ("system II")

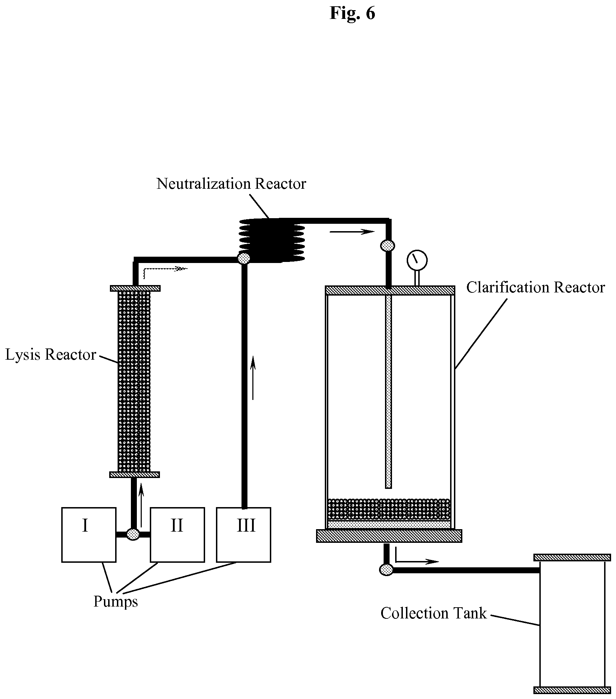

This experiment was carried out using the principle of the lab/pilot-scale system described in WO 2004/085643 applying semi-continuous clarification (system II). Compared to the original clarification reactor the distributor was adapted for the method with improved floatation/CO₂ release according to the method of the invention. Instead of a tube with slots reaching to the bottom of the clarification reactor (above the retention material) a tube with an open end and without perforations was used (Fig. 6). Due to the improved floatation effect by the attached gas bubbles, the flocs immediately floated upwards to the floc/liquid interface through the lower lysate phase. Therefore it was not necessary that the flocs entering the clarification reactor were distributed directly to the already present floc layer (level varying during process) by exiting through the perforations of the distributor initially used. Additionally a hollow spiral was built in to enable distribution of the floc-washing solution (at the end of the process) evenly over the whole floc layer/reactor diameter.

The experiment was carried out twice with 100 g biomass. In the first experiment (improved floatation) 0.05 M NaHCO₃ were added to the resuspension buffer. In the second experiment the standard parameters and the initial clarification reactor setup as described in WO 2004/085643 were used. The flow rate (influencing mixing and defining the contact time in the lysis and neutralization device) and other operational parameters were similar for both experiments. In both experiments the flow rate was adjusted to 20 mL/min for all 3 solutions/suspensions (resuspended biomass, lysis solution, neutralization solution) corresponding to a contact time of about 1.5 min for lysis and neutralization, respectively.

The lysate obtained by the setup with improved floatation was further processed, by concentrating it by hollow fiber ultrafiltration, conditioning it for binding on the subsequent hydrophobic interaction chromatography (HIC) step by mixing it with 4 M ammonium sulfate solution and filtration (HIC Load).

As a reference sample, an aliquot of the resuspended cells (without carbonate salt) equal to 1 g wet biomass was lysed and neutralized in a small tube according to the conventional lab-scale procedure, clarification being carried out by centrifugation (12.000 g). This sample was used to calculate the yield of the lab/pilot-scale process with improved floatation described here and to compare homogeneity (criterion for smoothness and quality). All samples were analyzed by HPLC (concentration, homogeneity, approximated purity). The results are summarized in Tab. 2.

**Tab. 2: Results of the continuous lysis/neutralization/clarification (system II) with improved floatation compared to the reference**

| Sample | Homogeneity (% ccc) | Purity (%) | Yield (%) |
|---|---|---|---|
| Reference | 90.3 | 6.4 | 100 |
| Lysate | 91.3 | 4.9 | - |
| HIC Load | 89.5 | 40.3 | 75.5* |

| | | | |
|---|---|---|---|
| * total yield up to HIC-Load (including all previous steps esp. lysis) | | | |

The results show that the homogeneity of the lysate obtained by the improved continuous method (system II clarification) is comparable to the reference. The yield and purity (up to HIC-Load) is comparable to that obtained with the continuous method without floatation. The subsequent HIC step worked as expected (comparable to the standard system with lysate obtained without improved floatation).

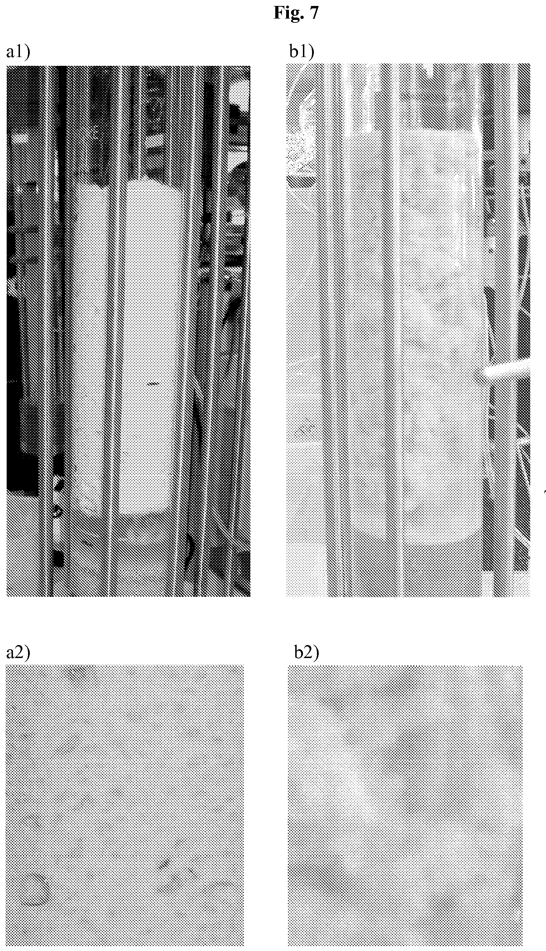

In Fig. 7 (a and b) the floatation of flocs (in the clarification reactor of WO 2004/085643) obtained by the method with and without CO₂ release are compared.

It is obvious that the method with CO₂ release resulted in a much more compact floc layer (a1) and zoomed in a2)) compared to the method without CO₂ (b1) and zoomed in b2)). This is of major importance for floc clarification, it is beneficial regarding capacity of the semi-continuous clarification reactor and consequently yield and it is a prerequisite for the completely continuous clarification system.

### Example 5

### Production of lysate by the method of the invention (with improved floatation; clarification mode system II) and subsequent purification.

100 g biomass were disintegrated as described in example 4 applying the method of the invention with CO₂ release and improved floatation. The collected lysate (3050 mL) was concentrated by hollow fiber ultrafiltration to 600 mL. In the next step the concentrated lysate was conditioned by mixing it with 4 M ammonium sulfate stock solution followed by filtration. An aliquot (575 mL) of the conditioned lysate was loaded on a HIC column of appropriate dimensions and eluted with a decreasing salt-gradient. The HIC pool was further purified by anion exchange chromatography (AEC) and size exclusion chromatography (SEC)and finally filtered by a 0.22 µm filter (drug substance). The results are summarized in Tab. 3 and 4.

The reference lysate was prepared as described in Example 4.

**Tab. 3: Product (pDNA) specific results of the continuous lysis/neutralization/clarification (system II) with improved floatation and subsequent purification steps compared to the reference.**

| Sample | Homogeneity (% ccc) | Yield (%) |
|---|---|---|
| Reference | 91.1 | 100 |
| Lysate | 92.0 | |
| Drug Substance | 94.6 | 47.3 |

**Tab. 4: Results of impurity analysis in the drug substance.**

| Impurity | Result |
|---|---|
| Endotoxins | < 0.480 EU/mL |
| | > 0.240 EU/mL |
| Genomic DNA | 1 ng/µg |
| RNA | < 1% |
| Protein | 1.47 µg/mL |

The results show that the pDNA homogeneity is not negatively influenced by the addition of a carbonate salt for CO₂ release during neutralization. Overall yield including also all (subsequent) purification steps was very good with nearly 50 % (upper end of overall yield achievable, when lysis/neutralization/clarification are carried out according to the routine method without CO₂-enhanced floatation described in WO 2004/085643), showing that the addition of the carbonate salt has no negative impact on purification, which is also confirmed by the low impurity content in the drug substance (comparable to results obtained for drug substance prepared according to the routine method) .

Similar results were obtained in an adequate experiment simulating the completely continuous clarification system (system I). In this experiment the floating flocs were continuously removed by sucking it off with a pump.

### Example 6

### Setting up the up-scaled clarification system of WO 2004/085643 for application as a system II (or III) device of the method of the invention and its utilization for the method of the invention.

The principle construction of the up-scaled clarification system was already described in WO 2004/085643. For the application of the method of the invention with improved floatation two main parts were redesigned. On the one hand instead of a slotted distributor a un-perforated tube, which reached (from the top, lateral) to the bottom of the clarification reactor (above the retention material) was used. On the other hand an additional washing device to perform washing (at the end of the process) from the top was installed (washing from the bottom was carried out as described in WO 2004/085643). This device was a rotating ball used for CIPing (cleaning in place), which was used to distribute the washing solution evenly over the flocs. The flow of the washing solution was thereby reduced compared to its use in CIP mode in order to avoid destruction of the flocs and possible redissolution of impurities

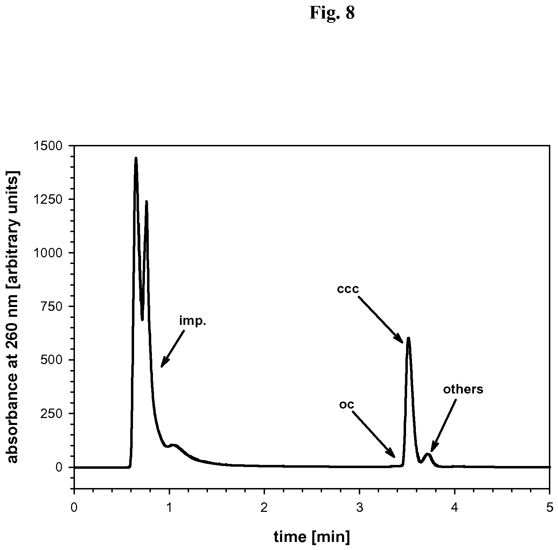

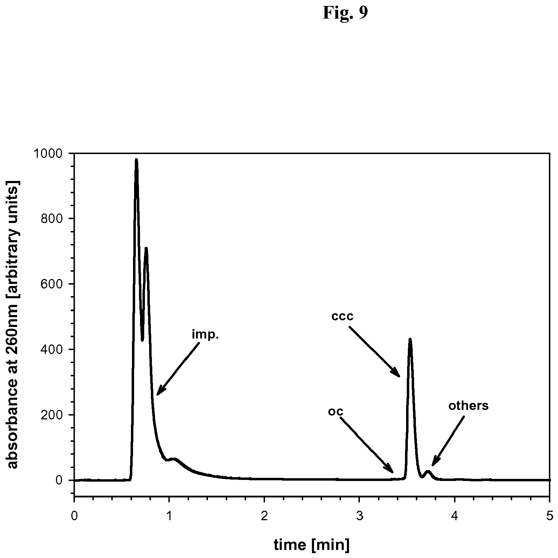

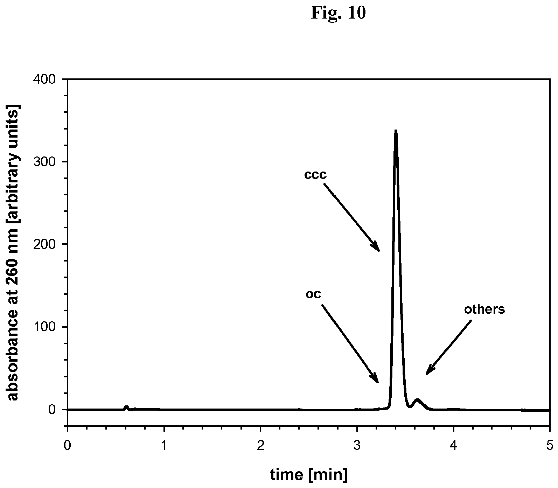

To show scalability of the novel improved clarification method with floc-floatation enhanced by attached CO₂ bubbles the adapted up-scaled system of WO 2004/085643 was used to prepare a clarified lysate processing 1.25 kg wet biomass. After resuspension of the previously frozen biomass, resulting in 13.5 L resuspension, and degassing the system, lysis, neutralization and clarification were carried out methodically as described in Examples 4 and 5. The pumps were adjusted to 0.75 L/min providing a contact/mixing time of about 1.5 min in the lysis and neutralization reactor. The resulting floc/lysate mixture was separated in the clarification reactor, where the majority of flocs were floating mediated by the attached CO₂ bubbles building a compact upper floc layer. At the end of the process after a first draining step the flocs retained by the retention material (glass beads with a diameter of 0.42 - 0.84 mm and 3 mm and polypropylene sinter plate) in the bottom of the clarification reactor were washed from both sides with a washing buffer at a flow rate of 3 L/min. Finally the flocs were drained by applying 0.5 bar over pressure (pressurized air). The obtained clarified lysate was further (stepwise) processed by the conditioning step (including filtration) and the subsequent chromatography steps (HIC, AEC and SEC). All samples were analyzed by HPLC (concentration, homogeneity, approximated purity). Figure 8 shows the analytical HPLC chromatogram of the reference lysate, Figure 9 the corresponding chromatogram of the lysate obtained in this experiment by the continuous system (system II clarification) and Figure 10 the analytical HPLC chromatogram of the SEC pool. The reference lysate was prepared as described in Example 4.

**Tab. 5: Product (pDNA) specific results of the continuous lysis/neutralization/clarification (system II) with improved floatation and subsequent purification steps compared to the reference obtained by the up-scaled system with adapted clarification reactor.**

| Sample | Homogeneity (% ccc) | Purity (%) | Yield (%) |
|---|---|---|---|
| Reference | 87.3 | 8.0 | 100 |
| Lysate | 91.9 | 6.3 | 70.1 |
| SEC Pool | 94.0 | 100 | 39.9* |

| | | | |
|---|---|---|---|
| * overall yield (compared to reference) including all previous steps | | | |

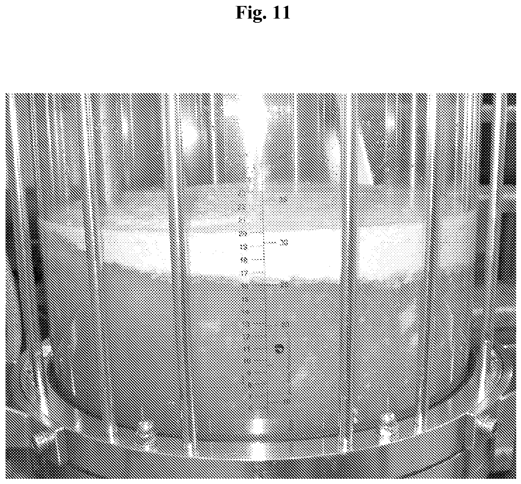

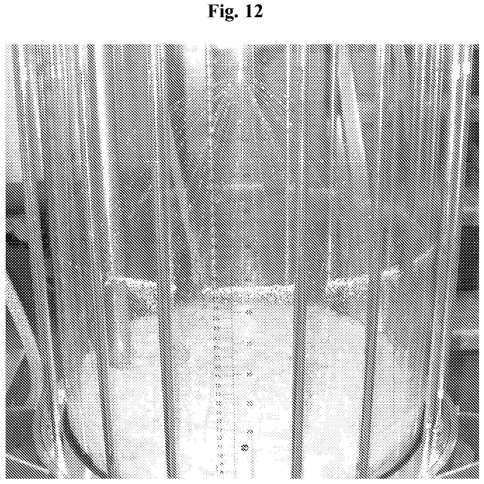

The product specific results confirmed that the method of the invention is scalable. All steps following lysis worked as expected confirming that the lysate obtained by the novel method of the invention (improved floatation by CO₂ release from carbonate salt can be further processed in the same way as a lysate obtained by the standard procedure (as described in of WO 2004/085643). Figure 11 shows the advantage of the improved floatation method in the up-scaled system - a compact floating floc-layer is obtained. Figure 12 shows the washing process by the CIP-ball at the top of the clarification reactor. This experiment was also repeated with 6-fold more biomass.

### Example 7

### Setting up a lab-scale system for application of the method of the invention in a complete continuous clarification mode (system I) and its utilization.

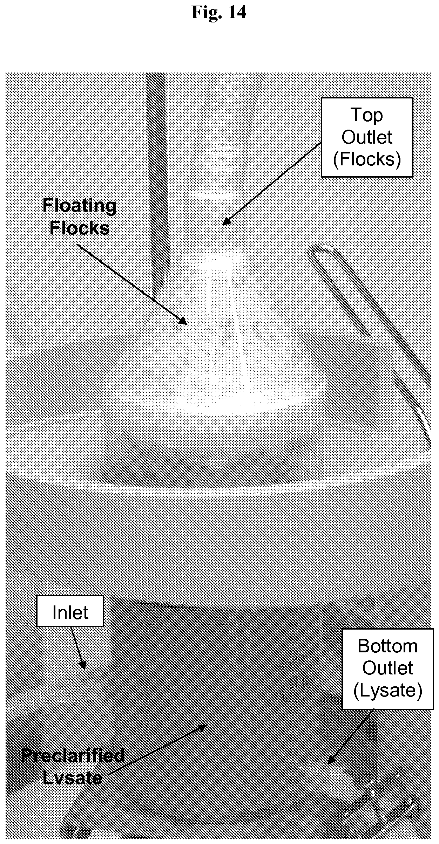

This experiment was carried out using the lysis and neutralization principle of the lab/pilot-scale system described in WO 2004/085643 extended by a novel setup for completely continuous (pre-)clarification (see Figure 13 and 14). Based on the principle design shown in Figure 13 the following development device (see Figure 14) for the continuous (pre-) clarification was constructed: a glass cylinder with a flat bottom, a diameter of 9.4 cm and a height of 19.4 cm was equipped with a lateral inlet in the middle of the cylinder and a lateral opposite outlet at the bottom of the cylinder. The inlet and the outlet port were connected to tubings of 8 mm inner diameter. The top of the cylinder was leakproof connected with a hopper as a tapering extension. This hopper tapered (from a diameter similar to the top of the cylinder) to a diameter of 22 mm within a length of about 65 mm. The tapered top end of the hopper was connected with a tubing of similar diameter representing the top outlet of the development device for continuous (pre-)clarification. The volume of the complete clarification device was about 1450 mL. If clarification is carried out in a semi-continuous mode utilizing a system II setup with a clarification device of similar volume (1450 mL) this volume would be sufficient to collect the flocs obtained from alkaline lysis/neutralization of about 100 g wet biomass.

The experiment was carried out with 500 g biomass resuspended in resuspension buffer containing 0.05 M NaHCO₃ (at pH 8). The flow rate was adjusted to 30 mL/min for all 3 solutions/suspensions (resuspended biomass, lysis solution, neutralization solution). At the beginning of the process the bottom outlet of the clarification device used to collect the (pre-)clarified lysate was closed and the clarification device filled with washing-buffer up to the inlet.

As observed in previous experiments the CO₂ release immediately started after contact of the lysed cell solution with the neutralization solution when the process was started and the small gas bubbles attached to the precipitate flocs which were concurrently generated. After passing the mixing distance (coiled tubing) the mixture of lysate and flocs of precipitate entered the novel clarification device. Thereby the clarification device was completely filled. Due to the gas bubbles attached to the flocs, the precipitate was directed upwards and separated from a lower clearer lysate phase which contained minimal flocs. When the flocs reached the top outlet, the bottom outlet was opened to recover the (pre-)clarified lysate. Since the bottom outlet was only partly opened the flow rate of the lysate exiting via the bottom outlet was lower than the flow rate of the entering mixture. Thus the flocs separated in the upper part of the clarification device and containing minimal inter-floc lysate, further reduced by the tapering top of the device, were forced through the top outlet and collected via a tubing in a subsequently located sieve. Residual lysate was collected in a container below the sieve. The degree of opening of the bottom outlet was manually adjusted in a way to keep the interface lysate/flocs in the device approximately in mid-height of the device. The collected (pre-)clarified lysate was fed into a retention material containing clarification device described in WO 2004/085643 for fine clarification. Instead of the clarification device of WO 2004/085643 also conventional depth filters could have been used for fine-clarification prior to purification by chromatography. If a conditioning step such as ammonium sulfate precipitation is planed prior chromatography the lysate could be processed without fine-clarification. The fine-clarified lysate was collected at the exit of the clarification device of WO 2004/085643 and combined with the floc-drainage and floc-washing fractions. At the end of the process the feed to the clarification device was stopped and the lower lysate phase left in the device was recovered via the bottom outlet (till the flocs reached the outlet). Then the bottom outlet was closed and the feed started again with washing buffer as long as the bulk of flocs left in the clarification device was forced through the top outlet.

In the described experiment 500 g wet biomass could be processed without any limitation. This is 5-times more compared to the amount which could have been processed in a size-comparable semi-continuous system of WO 2004/085643. Since it could be shown that the novel (pre-)clarification system was not limited regarding amount of biomass processed, the system enables processing of much more biomass than the 500 g applied in this experiment. Separation/(pre-)clarification of flocs and lysate by the fully continuous (pre-)clarification system worked very satisfactory. The lysate collected at the bottom outlet contained only minimal residual small flocs and the flocs exiting via the top outlet were compact, containing only minimal residual inter-floc lysate. The experiment showed that an infinite amount of biomass can be processed (including (pre-)clarification) by the method and device of the invention. The volume of lysate collected at the end of processing 500 g wet biomass including the wash fraction was about 16920 mL. The lysate was analyzed by HPLC for pDNA concentration and homogeneity as well as for approximated purity (last two as criteria for smoothness and quality) and the results were compared with the results of the reference lysate, which was prepared as described in Example 4 . The collected lysate contained overall about 1.4 g total pDNA.

The results are summarized in Tab. 6.

**Tab. 6: Product (pDNA) specific results of the continuous lysis/neutralization and fully continuous clarification mediated by improved floatation (system I) compared to the reference.**

| Sample | Homogeneity (% ccc) | Purity (%) | Yield (%) |
|---|---|---|---|
| Reference | 76.8 | 9.3 | 100 |
| Lysate | 76.0 | 9.5 | 89.0* |

| | | | |
|---|---|---|---|
| *compared to reference | | | |

The results show that the homogeneity and estimated purity of the lysate obtained by the improved fully continuous method is comparable to the reference. The yield with nearly 90 % was very good and better compared to the previous experiments, which is of major economic importance.

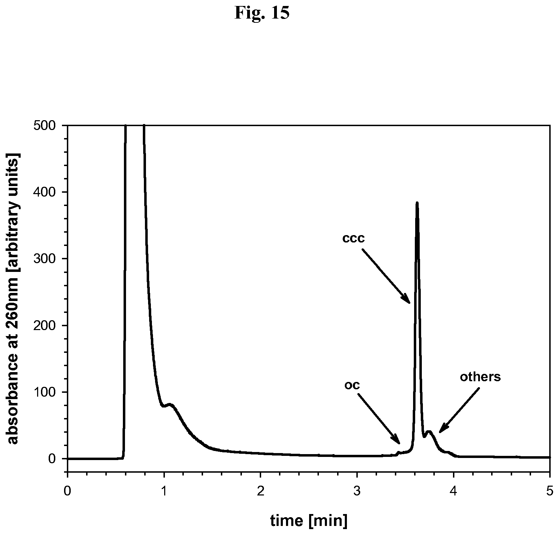

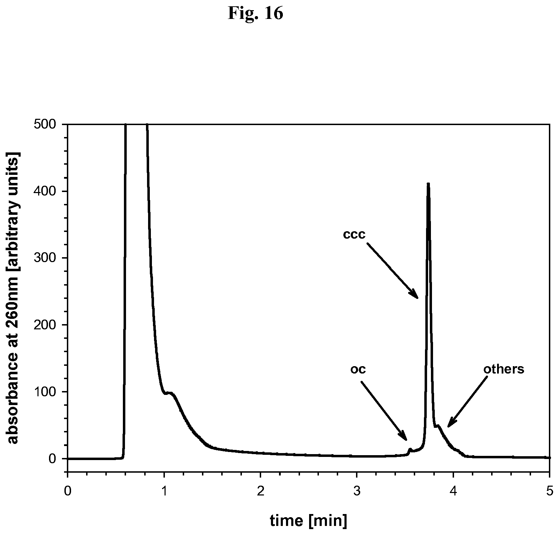

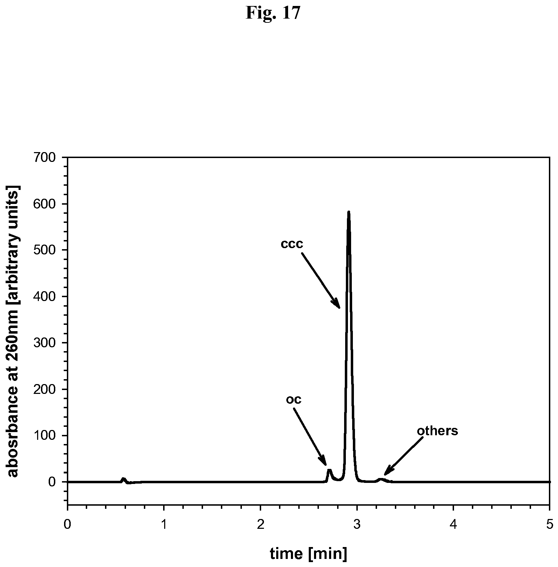

In Fig. 15 the analytical HPLC chromatogram of a reference lysate (without CO₂ release) is shown and can be compared with the analytical HPLC chromatogram (Fig. 16) of the lysate obtained in this experiment utilizing fully continuous clarification mode system I. Both chromatograms are comparable and show similar peak pattern, confirming that the novel method utilizing the novel (pre-)clarification device can be applied without negatively influencing lysate/pDNA quality maintaining homogeneity and estimated purity. In Fig. 17 the analytical HPLC chromatogram of the SEC Pool as last purification step of the lysate obtained in this experiment is shown. SEC was applied after concentrating the lysate, conditioning (ammonium sulfate precipitation and filtration) and HIC- and AEC-purification. The pDNA homogeneity in the SEC-Pool was 94.3 % showing that the lysate obtained by the method and device of the invention in this experiment could be successfully purified reaching a high final ccc pDNA-rate.

### Example 8

### Prototype lab-scale system for application of the method of the invention in a complete continuous clarification mode (system I) and its utilization.

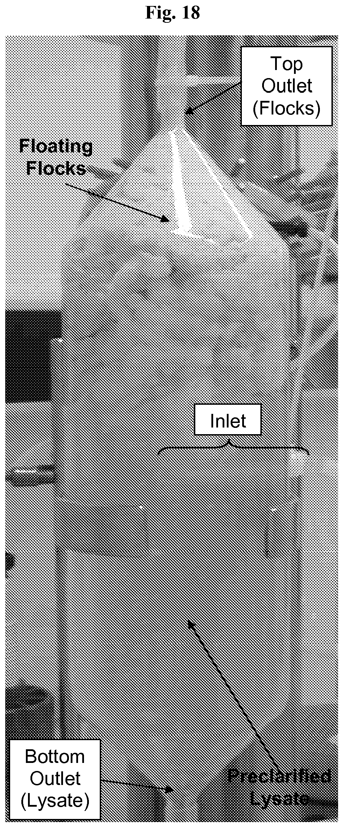

This experiment was carried out using the lysis and neutralization principle of the lab/pilot-scale system as described in WO 2004/085643 with a novel setup for completely continuous (pre-)clarification. This prototype (see Figure 18) contained all necessary parts for a continuous process. For the continuous (pre-)clarification process a prototype according to the design shown in Figure 13 was used. Compared to the development device as described in example 7, the novel glass cylinder of this prototype tapered at the bottom and at the top. Furthermore, the inlet was located laterally in the middle between the bottom and the top outlet and ended radially in the middle of the cylinder. The inlet and the outlets were connected to tubings of 8 mm inner diameter.

Experiments were carried out in a similar way as described in example 7 with up to 1000 g biomass using 0.05 to 0.1 M carbonate salt in the resuspension solution. The flocks floated very well separating from the lower clearer lysate phase and built a compact layer at the top part of the device resulting in an even better performance of the clarification process. The flocks were forced out through the top outlet with minimal residual lysate in between. The tapering bottom supports maximal recovery of (pre-) clarified lysate. The homogeneity (quality) of the pDNA in the lysate was similar to the reference and yield was about 90 %. No limitations regarding capacity were observed.

## Claims

1. A method for producing DNA or RNA that is not secreted by the host cells, comprising the steps of
a) cultivating host cells to produce DNA or RNA,
b) disintegrating the cells by alkaline lysis,
c) neutralizing the lysate obtained in step b), whereby a precipitate is formed,
d) separating the cleared lysate from the precipitate obtained in step c),
e) purifying DNA or RNA,
**characterised in that**
a carbonate salt is added in at least one of step a - d), whereby due to the neutralization in step c) CO₂ is released and wherein in step d) the precipitate and the lysate are allowed to separate in a clarification device.

2. The method according to claim 1, wherein the cleared lysate obtained in step d) leaves the clarification reactor/device through an outlet at the bottom.

3. The method according to claim 1, wherein the carbonate salt is added in step a).

4. The method according to claim 1, wherein the carbonate salt is added in step b).

5. The method according to claim 1, wherein the carbonate salt is added in step c).

6. The method according to one of claims 1 to 5, wherein the calculated theoretical carbonate concentration in the resulting lysate-floc mixture with attached CO₂ bubbles is in the range of about 0.003 to about 0.35 M.

7. The method according to claim 6, wherein the calculated theoretical carbonate concentration in the resulting lysate-floc mixture with attached CO₂ bubbles is in the range of about 0.005 to about 0.05 M.

8. The method according to one of claims 1 to 7, wherein the carbonate salt is NaHCO₃.

9. The method according to claim 1, wherein step d) is operated in a semi-continuous or continuous mode.

10. The method of claim 1 in a continuous mode, wherein step d) uses a device comprising a container, which is equipped with
a) two outlets, wherein one is positioned at the top of the container and the other one at the bottom of the container, and
b) an inlet between the two outlets.

11. The method according to claim 10, wherein the container is connected with an additional drain and/or wash unit.

12. The method according to claim 1, wherein at least one step selected from steps b) to e) is operated in a continuous mode.

13. The method according to claim 1, wherein at least a combination of two steps selected from steps b) to e) is operated in a continuous mode by connecting the two or more individual steps.

14. The method according to claim 12 or 13, wherein in addition step a) is operated in a continuous mode by being connected to step b).

15. The method according to one of claims 12 to 14, wherein at least one step is operated in an automated mode.

16. The method of claim 1, wherein a washing step of the precipitate is inserted between step d) and step e).

17. The method according to claim 16, wherein the washing step is conducted in an additional drain/wash unit of claim 11.

18. The method according to claim 1, wherein a concentration and/or a conditioning step is inserted between step d) and step e).

19. The method according to claim 18, wherein said concentration step takes place before said conditioning step.

20. The method of claim 1, wherein the lysate of step d) contains the DNA or RNA.

21. The method of claim 1, wherein the cell mass obtained in step a) is cryo-pelleted and thereafter resuspended.

22. Use of a device for carrying out step d) in the method according to claim 1 in a semi-continuous-mode, comprising a container which is equipped with
a) a retention layer in its lower part,
b) an inlet at a position above the retention layer,
c) an outlet underneath the retention layer, and
d) one or more distribution means that reach to the surface of the retention layer and evenly and gently distribute a mixture of precipitate and lysate as obtained upon alkaline lysis and neutralization into the container.

23. The use according to claim 22, comprising in addition means for supply of compressed gas.

## Patentansprüche

1. Verfahren zur Herstellung von DNA oder RNA die nicht von den Wirtszellen ausgeschieden wird, umfassend die Schritte
a) Kultivieren von Wirtszellen zur Herstellung von DNA oder RNA,
b) Zersetzen der Zellen durch alkalische Lyse,
c) Neutralisieren des in Schritt b) erhaltenen Lysats, wodurch ein Niederschlag gebildet wird,
d) Abtrennen des geklärten Lysats von dem in Schritt c) erhaltenen Niederschlag,
e) Reinigen der DNA oder RNA,
**dadurch gekennzeichnet, dass**
ein Carbonatsalz in mindestens einem der Schritte a - d) zugegeben wird, wobei aufgrund der Neutralisation in Schritt c) CO₂ freigesetzt wird und wobei sich in Schritt d) der Niederschlag und das Lysat in einer Klärvorrichtung trennen können.

2. Verfahren nach Anspruch 1, wobei das in Schritt d) erhaltene geklärte Lysat den Klärungsreaktor/die Klärvorrichtung durch einen Auslass am Boden verlässt.

3. Verfahren nach Anspruch 1, wobei das Carbonatsalz in Schritt a) zugegeben wird.

4. Verfahren nach Anspruch 1, wobei das Carbonatsalz in Schritt b) zugegeben wird.

5. Verfahren nach Anspruch 1, wobei das Carbonatsalz in Schritt c) zugegeben wird.

6. Verfahren nach einem der Ansprüche 1 bis 5, wobei die berechnete theoretische Carbonatkonzentration in der resultierenden Lysat-Flocken-Mischung mit gebundenen CO₂-Blasen im Bereich von etwa 0,003 bis etwa 0,35 M liegt.

7. Verfahren nach Anspruch 6, wobei die berechnete theoretische Carbonatkonzentration in der resultierenden Lysat-Flocken-Mischung mit gebundenen CO₂-Blasen im Bereich von etwa 0,005 bis etwa 0,05 M liegt.

8. Verfahren nach einem der Ansprüche 1 bis 7, wobei das Carbonatsalz NaHCO₃ ist.

9. Verfahren nach Anspruch 1, wobei der Schritt d) in einem halbkontinuierlichen oder kontinuierlichen Modus betrieben wird.

10. Verfahren nach Anspruch 1 in einem kontinuierlichen Modus, wobei Schritt d) eine Vorrichtung verwendet, die einen Behälter umfasst, der ausgestattet ist mit
a) zwei Auslässen, wobei einer am Kopfende des Behälters und der andere am Boden des Behälters positioniert ist, und
b) einen Einlass zwischen den zwei Auslässen.

11. Verfahren nach Anspruch 10, wobei der Behälter mit einer zusätzlichen Ablauf- und/oder Wascheinheit verbunden ist.

12. Verfahren nach Anspruch 1, wobei mindestens ein aus den Schritten b) bis e) ausgewählter Schritt in einem kontinuierlichen Modus betrieben wird.

13. Verfahren nach Anspruch 1, wobei mindestens eine Kombination von zwei Schritten, die aus den Schritten b) bis e) ausgewählt werden, in einem kontinuierlichen Modus betrieben wird, indem die zwei oder mehr Einzelschritte miteinander verbunden werden.

14. Verfahren nach Anspruch 12 oder 13, wobei zusätzlich Schritt a) in einem kontinuierlichen Modus betrieben wird, indem er mit Schritt b) verbunden wird.

15. Verfahren nach einem der Ansprüche 12 bis 14, wobei mindestens ein Schritt in einem automatisierten Modus ausgeführt wird.

16. Verfahren nach Anspruch 1, wobei ein Waschschritt des Niederschlags zwischen Schritt d) und Schritt e) eingefügt wird.

17. Verfahren nach Anspruch 16, wobei der Waschschritt in einer zusätzlichen Ablauf-/Wascheinheit nach Anspruch 11 durchgeführt wird.

18. Verfahren nach Anspruch 1, wobei ein Konzentrations- und/oder ein Konditionierungsschritt zwischen Schritt d) und Schritt e) eingefügt wird.

19. Verfahren nach Anspruch 18, wobei der Konzentrationsschritt vor dem Konditionierungsschritt stattfindet.

20. Verfahren nach Anspruch 1, wobei das Lysat von Schritt d) die DNA oder RNA enthält.

21. Verfahren nach Anspruch 1, wobei die in Schritt a) erhaltene Zellmasse kryopelletiert und danach resuspendiert wird.

22. Verwendung einer Vorrichtung zur Durchführung von Schritt d) in dem Verfahren gemäß Anspruch 1 in einem semikontinuierlichen Modus, umfassend einen Behälter, der ausgestattet ist mit
a) einer Retentionsschicht in seinem unteren Teil,
b) einem Einlass an einer Stelle über der Retentionsschicht,
c) einem Auslass unter der Retentionsschicht und
d) ein oder mehrere Verteilungsmittel, die an die Oberfläche der Retentionsschicht reichen und gleichmäßig und behutsam eine Mischung von Niederschlag und Lysat, wie durch alkalische Lyse und Neutralisation erhalten, in den Behälter verteilen.

23. Verwendung nach Anspruch 22, umfassend zusätzlich Mittel zur Zufuhr von komprimiertem Gas.

## Revendications

1. Méthode pour produire un ADN ou un ARN qui n'est pas sécrété par les cellules hôtes, comprenant les étapes suivantes
a) la mise en culture de cellules hôtes pour produire un ADN ou un ARN,
b) la désintégration des cellules par lyse alcaline,
c) la neutralisation du lysat obtenu dans l'étape b), moyennant quoi un précipité est formé,
d) la séparation du lysat clarifié du précipité obtenu dans l'étape c),
e) la purification d'un ADN ou d'un ARN
**caractérisée en ce que**
un sel de carbonate est ajouté dans au moins l'une des étapes a) à d), au moyen duquel en raison de la neutralisation dans l'étape c) du CO₂ est libéré et dans laquelle dans l'étape d) le précipité et le lysat sont laissés à se séparer dans un dispositif de clarification.

2. Méthode selon la revendication 1, dans laquelle le lysat clarifié obtenu dans l'étape d) quitte le réacteur/dispositif de clarification par une sortie au niveau du fond.

3. Méthode selon la revendication 1, dans laquelle le sel de carbonate est ajouté dans l'étape a).

4. Méthode selon la revendication 1, dans laquelle le sel de carbonate est ajouté dans l'étape b).

5. Méthode selon la revendication 1, dans laquelle le sel de carbonate est ajouté dans l'étape c).

6. Méthode selon l'une des revendications 1 à 5, dans laquelle la concentration théorique calculée en carbonate dans le mélange de lysat-floc résultant avec des bulles de CO₂ attachées est située dans la plage allant d'environ 0,003 à environ 0,35 M.

7. Méthode selon la revendication 6, dans laquelle la concentration théorique calculée en carbonate dans le mélange de lysat-floc résultant avec des bulles de CO₂ attachées est située dans la plage allant d'environ 0,005 à environ 0,05 M.

8. Méthode selon l'une des revendications 1 à 7, dans laquelle le sel de carbonate est NaHCO₃.

9. Méthode selon la revendication 1, dans laquelle l'étape d) est effectuée dans un mode semi-continu ou continu.

10. Méthode selon la revendication 1 dans un mode continu, dans laquelle l'étape d) utilise un dispositif comprenant un récipient, qui est équipé
a) de deux sorties, dans laquelle une est positionnée en haut du récipient et l'autre au fond du récipient, et
b) d'une entrée entre les deux sorties.

11. Méthode selon la revendication 10, dans laquelle le récipient est connecté à une unité de vidange et/ou de lavage supplémentaire.

12. Méthode selon la revendication 1, dans laquelle au moins une étape sélectionnée parmi les étapes b) à e) est effectuée dans un mode continu.

13. Méthode selon la revendication 1, dans laquelle au moins une combinaison de deux étapes sélectionnées parmi les étapes b) à e) est effectuée dans un mode continu en reliant les deux ou plus de deux étapes individuelles.

14. Méthode selon la revendication 12 ou 13, dans laquelle de plus l'étape a) est effectuée dans un mode continu en étant reliée à l'étape b).

15. Méthode selon l'une des revendications 12 à 14, dans laquelle au moins une étape est effectuée dans un mode automatisé.

16. Méthode selon la revendication 1, dans laquelle une étape de lavage du précipité est insérée entre l'étape d) et l'étape e).

17. Méthode selon la revendication 16, dans laquelle l'étape de lavage est menée dans une unité de vidange/lavage supplémentaire de la revendication 11.

18. Méthode selon la revendication 1, dans laquelle une étape de concentration et/ou de conditionnement est insérée entre l'étape d) et l'étape e).

19. Méthode selon la revendication 18, dans laquelle ladite étape de concentration a lieu avant ladite étape de conditionnement.

20. Méthode selon la revendication 1, dans laquelle le lysat de l'étape d) contient l'ADN ou l'ARN.

21. Méthode selon la revendication 1, dans laquelle la masse cellulaire obtenue dans l'étape a) est cryo-agglomérée et par la suite remise en suspension.

22. Utilisation d'un dispositif pour mettre en oeuvre l'étape d) dans la méthode selon la revendication 1 dans un mode semi-continu, comprenant un récipient qui est équipé
a) d'une couche de rétention dans sa partie inférieure,
b) d'une entrée au niveau d'une position au-dessus de la couche de rétention,
c) d'une sortie sous la couche de rétention, et
d) d'un ou de plusieurs moyens de distribution qui atteignent la surface de la couche de rétention et répartissent uniformément et doucement un mélange de précipité et de lysat tel qu'obtenu après une lyse alcaline et une neutralisation dans le récipient.

23. Utilisation selon la revendication 22, comprenant en plus des moyens d'alimentation en gaz comprimé.
